(19) Europäisches Patentamt
European Patent Office
Office européen des brevets

(11) EP 0 593 997 B1

(12) **EUROPÄISCHE PATENTSCHRIFT**

(45) Veröffentlichungstag und Bekanntmachung des Hinweises auf die Patenterteilung: **09.04.1997 Patentblatt 1997/15**

(21) Anmeldenummer: **93116305.9**

(22) Anmeldetag: **08.10.1993**

(51) Int. Cl.$^6$: **C07C 25/18**, C09K 19/30, C09K 19/34, C07C 43/192, C07C 255/46, C07D 213/24, C07D 239/26, C07D 319/06, G02F 1/13

(54) **Fluorsubstituierte Phenyl-Cyclohexylacetylene und sie enthaltende flüssigkristalline Gemische**

Fluor substituted phenyl-cyclohexyl acetylenes and liquid crystals mixtures containing them

Phenyl-cyclohexyl acétylènes substitués par du fluor et mélanges de cristaux liquides les contenant

(84) Benannte Vertragsstaaten: **CH DE FR GB IT LI NL**

(30) Priorität: **23.10.1992 CH 3309/92**
**03.11.1992 CH 3425/92**
**16.03.1993 CH 789/93**

(43) Veröffentlichungstag der Anmeldung: **27.04.1994 Patentblatt 1994/17**

(73) Patentinhaber: **MERCK PATENT GmbH**
**64293 Darmstadt (DE)**

(72) Erfinder:
- **Buchecker, Richard**
  **CH-8008 Zürich (CH)**
- **Schadt, Martin**
  **CH-4411 Seltisberg (CH)**

(56) Entgegenhaltungen:
**WO-A-88/07523** **DE-A- 4 105 742**

EP 0 593 997 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Rank Xerox (UK) Business Services
2.14.2/3.4

## Beschreibung

Die vorliegende Erfindung betrifft neue, fluorsubstituierte Phenyl-Cyclohexylacetylene, deren Herstellung, flüssigkristalline Gemische, die solche Verbindungen enthalten, sowie die Verwendung dieser Verbindungen bzw. Gemische für elektro-optische Zwecke.

Flüssige Kristalle werden vor allem als Dielektrika in Anzeigevorrichtungen verwendet, da die optischen Eigenschalten solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gas/Wirt-Zellen, TN-Zellen mit verdrillt nematischer ("twisted nematic") Struktur, STN-Zellen ("super twisted nematic"), SBE-Zellen ("super birefringence effect") und OMI-Zellen ("optical mode interference"). Fur Displays mit hohem Informationsgehalt sind in letzter Zeit neben den passiv angesteuerten, multiplexierten, besonders die aktiv angesteuerten, z.B. TFT-Zellen ("thin film transistor") wichtig geworden. Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische, photochemische und thermische Stabilität und eine gute Stabilität gegenüber elektrischen Feldern besitzen. Ferner sollten sie über einen möglichst breiten Bereich eine geeignete Mesophase aufweisen (beispielsweise für die oben genannten Zellen eine nematische bzw. eine cholesterische Phase), aber trotzdem ausreichend niedere Viskosität besitzen und in den Zellen kurze Ansprechzeiten, tiefe Schwellenspannungen und einen hohen Kontrast ermöglichen. Weitere Eigenschaften, wie die elektrische Leitfähigkeit, die dielektrische Anisotropie und die optische Anisotropie, müssen je nach Anwendungsgebiet und Zellentyp unterschiedlichen Anforderungen genügen. Beispielsweise sollten Materialien für die Zellen mit verdrillt nematischer Struktur eine möglichst hohe positive dielektrische Anisotropie und gleichzeitig eine möglichst geringe Leitfähigkeit aufweisen. Diese letztere Eigenschaft ist vorallem für TFT-Zellen von besonderer Wichtigkeit. Es werden daher Komponenten gesucht, die sich durch eine möglichst hohe dielektrische Anisotropie bei gleichzeitig möglichst geringer Leitfähigkeit auszeichnen. **In WO-A-88 07523 werden Acetylen Derivate und in DE-A-4 105 742 werden Tolane Derivate als Flüssigkristall-Komponenten beschrieben.**

Gegenstand der **vorliegenden** Erfindung sind Verbindungen der allgemeinen Formel

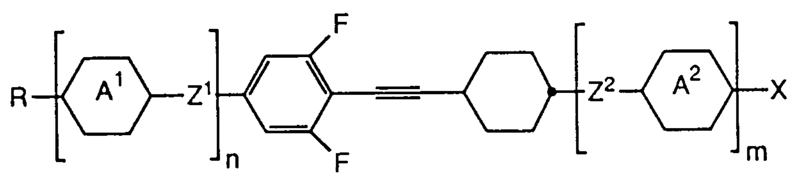

I

worin

- R Alkyl, Alkoxy, Alkenyl oder Alkenyloxy mit 1 bzw. 2 bis 12 Kohlenstoffatomen bedeutet, in welchen eine $CH_2$-Gruppe durch Sauerstoff und/oder eines oder mehrere Wasserstoffatome durch Fluoratome ersetzt sein können, mit der Massgabe, dass zwei Sauerstoffatome nicht direkt benachbart sind;
- $A^1$ 1,4-Phenylen, Pyridin-2,5-diyl, Pyrimidin-2,5-diyl, trans-1,4-Cyclohexylen oder trans 1,3-Dioxan-2,5-diyl darstellt;
- $A^2$ trans-1,4-Cyclohexylen oder gegebenenfalls mit Fluor substituiertes 1,4-Phenylen bedeutet;
- $Z^1$ eine einfache Kovalenzbindung, $-CH_2CH_2-$, $-OCH_2-$, $-CH_2O-$, $-(CH_2)_4-$, $-O(CH_2)_3-$, $-(CH_2)_3O-$ oder, falls Ring $A^1$ einen gesättigten Ring darstellt, auch die trans-Form von $-CH=CH(CH_2)_2-$ oder $-CH=CHCH_2O-$ bedeutet;
- $Z^2$ eine einfache Kovalenzbindung, $-CH_2CH_2-$, $-OCH_2-$, $-CH_2O-$, $-(CH_2)_4-$, $-O(CH_2)_3-$, $-(CH_2)_3O-$, oder die trans-Form von $-CH=CH(CH_2)_2-$ oder $-CH=CHCH_2O-$ bedeutet;
- n 0 oder 1 bedeutet;
- m 0 oder 1 bedeutet, mit der Massgabe, dass $n+m \leq 1$ ist; und
- X Cyano, $-CF_3$, $-OCF_3$, $-OCHF_2$, $-CH=CF_2$, Alkyl, Alkenyl, Alkoxy, Alkenyloxy oder Alkoxyalkyl mit 1 bzw. 2 bis 6 Kohlenstoffatomen, oder an trans-1,4-Cyclohexylen auch $-CH=CHF$ oder $-CH=CHCl$, oder an 1,4-Phenylen auch Fluor oder Chlor bedeutet.

Die erfindungsgemässen Verbindungen sind Flüssigkristalle mit ausgeprägter nematischer Phase, vergleichsweise hoher dielektrischer Anisotropie, relativ niedriger Rotationsviskosität, und führen zu vergleichsweise niedrigen Schwellenspannungen und kurzen Ansprechzeiten. Zudem ist der Klärpunkt trotz doppelter lateraler Substitution überraschend hoch bei vergleichsweise niedrigem Schmelzpunkt und kleiner Schmelzenthalpie. Durch die geeignete Wahl eines gesättigten oder aromatischen Ringes für $A^1$ oder $A^2$ kann die optische Anisotropie wunschgemäss erniedrigt

oder weiter erhöht werden. Die dielektrische Anisotropie kann zudem durch geeignete Wahl des Substituenten X beeinflusst werden.

Die erfindungsgemässen Verbindungen sind in Mischungen sehr gut und in breiten Konzentrationsbereichen löslich. Sie eignen sich besonders zur Anwendung in Mischungen, die bei tiefer Schwellenspannung eine tiefe Leitfähigkeit und gleichzeitig eine geeignete - je nach Bedarf - hohe, beziehungsweise niedrige optische Anisotropie aufweisen sollten, beispielsweise für TN-, STN- oder TFT-Zellen.

Der Ausdruck "gesättigter Ring" umfasst im Rahmen der vorliegenden Erfindung trans-1,4-Cyclohexylen oder trans-1,3-Dioxan-2,5-diyl.

In den Verbindungen der Formel I, worin $A^1$ einen heterocyclischen Ring wie Pyridin-2,5-diyl, Pyrimidin-2,5-diyl oder trans-1,3-Dioxan-2,5-diyl bedeutet, sind die Heteroatome im Ring ausschliesslich so angeordnet, dass sie eine zur Verknüpfungsstelle mit $Z^1$ benachbarte Stellung einnehmen.

Der Ausdruck "unsubstituiertes oder gegebenenfalls mit Fluor substituiertes 1,4-Phenylen" umfasst im Zusammenhang mit Ring $A^2$ unsubstituierte, mono- oder difluorierte 1,4-Phenylen Ringe.

Das Brückenglied $Z^1$ bedeutet bevorzugt eine einfache Kovalenzbindung, $-CH_2CH_2-$, $-OCH_2-$ oder $-CH_2O-$, besonders bevorzugt jedoch eine einfache Kovalenzbindung oder $-CH_2CH_2-$.

Das Brückenglied $Z^2$ bedeutet bevorzugt eine einfache Kovalenzbindung oder $-CH_2CH_2-$.

In den Verbindungen der allgemeinen Formel I, worin X Alkyl, Alkoxy oder Alkoxyalkyl bedeutet, werden geradkettige Reste mit 1 bzw. 2 bis 6 Kohlenstoffatomen bevorzugt, insbesondere werden Reste mit 1 bzw. 2 bis 3 Kohlenstoffatomen bevorzugt, wie beispielsweise Methyl, Aethyl, Propyl, Methoxy, Aethoxy, Propyloxy, Methoxymethyl, Aethoxymethyl, Methoxyethyl und dergleichen

In den Verbindungen der allgemeinen Formel I, worin X Alkenyl oder Alkenyloxy bedeutet, werden geradkettige Reste mit 2 bis 6 Kohlenstoffatomen bevorzugt. Bevorzugte Alkenyl Reste sind diejenigen, worin die Doppelbindung endständig ist oder E-konfiguriert ist und sich an C(3), oder an einem trans-1,4-Cyclohexylen Ring auch an C(1) befindet. Bevorzugte Alkenyloxy Reste sind diejenigen mit einer E-konfigurierten Doppelbindung an C(2) oder solche mit einer endständigen Doppelbindung. Beispiele solcher Reste sind Vinyl, 1E-Propenyl, 1E-Butenyl, 1E-Pentenyl, 1E-Hexenyl, 3-Butenyl, 3E-Pentenyl, 3E-Hexenyl, 4-Pentenyl, 5-Hexenyl, Allyloxy, 2E-Butenyloxy, 3-Butenyloxy und dergleichen

Der Ausdruck "Alkyl, Alkoxy, Alkenyl oder Alkenyloxy mit 1 bzw. 2 bis 12 Kohlenstoffatomen, in welchen eine $CH_2$-Gruppe durch Sauerstoff und/oder eines oder mehrere Wasserstoffatome durch Fluoratome ersetzt sein können, mit der Massgabe, dass zwei Sauerstoffatome nicht direkt benachbart sind" umfasst im Rahmen der vorliegenden Erfindung geradkettige oder verzweigte, gegebenenfalls chirale Alkyl, Alkoxy, Alkenyl, Alkenyloxy, Alkoxyalkyl, Alkenyloxyalkyl und Alkyloxyalkenyl Reste mit 1 bzw. 2 bis 12 Kohlenstoffatomen, welche einfach oder mehrfach mit Fluor substituiert sein können. Bevorzugte Reste sind unsubstituierte, geradkettige Reste mit 1 bzw. 2 bis 6 Kohlenstoffatomen. Bevorzugte Alkenylreste sind diejenigen, worin die Doppelbindung E-konfiguriert ist und sich an C(3) oder, falls Ring $A^1$ einen gesättigten Ring darstellt, auch an C(1) befindet oder aber endständig ist. Bevorzugte Alkenyloxyreste sind diejenigen mit einer E-konfigurierten Doppelbindung an C(2) oder solche mit einer endständigen Doppelbindung. Beispiele solcher Reste sind Methyl, Aethyl, Propyl, Butyl, Pentyl, Hexyl, Methoxy, Aethoxy, Propyloxy, Butyloxy, Pentyloxy, Hexenyloxy, Vinyl, 1E-Propenyl, 1E-Butenyl, 1E-Pentenyl, 1E-Hexenyl, 3-Butenyl, 3E-Pentenyl, 3E-Hexenyl, 4-Pentenyl, 5-Hexenyl, Allyloxy, 2E-Butenyloxy, 3-Butenyloxy, Methoxymethyl, Aethoxymethyl, Propyloxymethyl, Allyloxymethyl, Methoxyäthyl, Aethoxyäthyl, Propyloxyäthyl, Methoxypropyl, Aethoxypropyl, Methoxy-1E-propenyl, Aethoxy-1E-propenyl und dergleichen.

Besonders bevorzugte Untergruppen der Verbindungen der Formel I sind Verbindungen der allgemeinen Formeln

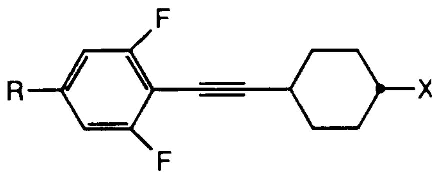

I-1

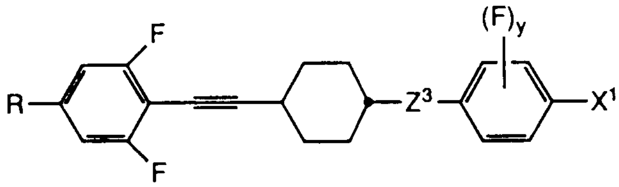

I-2

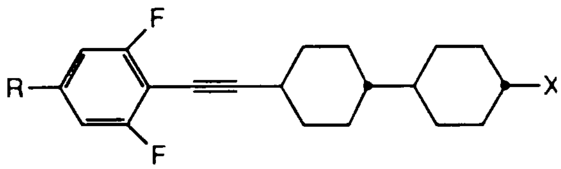

I-3

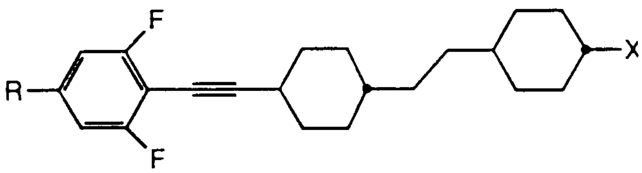

I-4

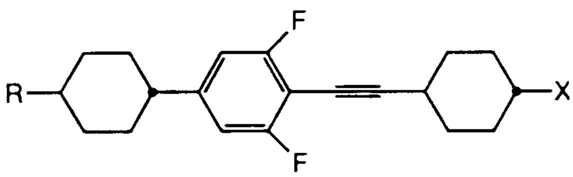

I-5

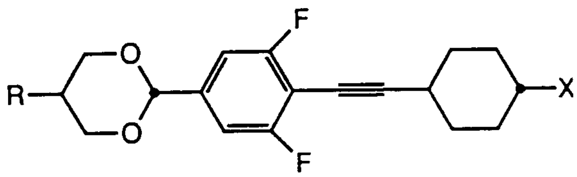

I-6

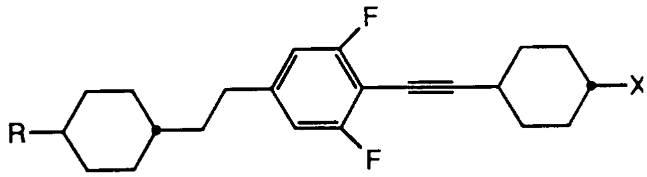

I-7

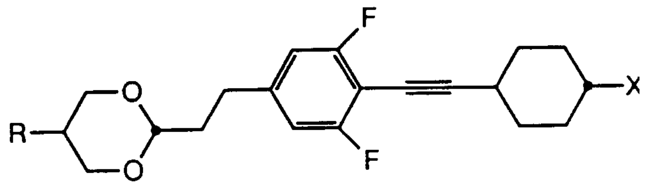

I-8

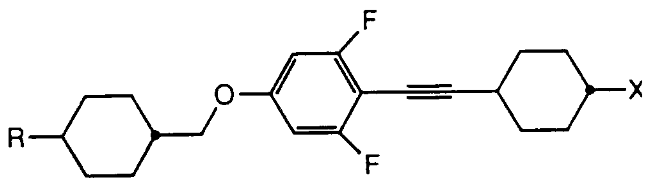

I-9

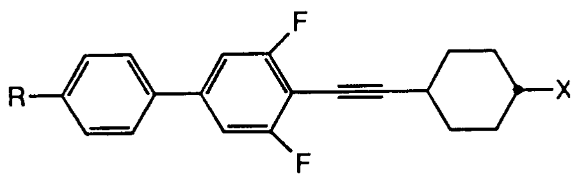

I-10

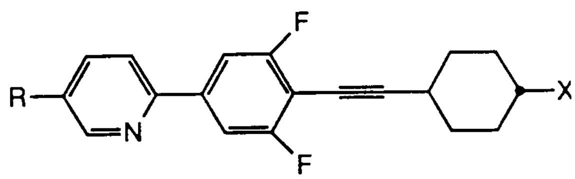

I-11

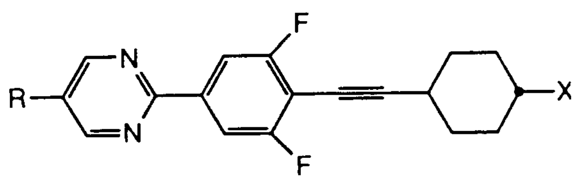

I-12

worin

- R Alkyl, Alkoxy, Alkenyl oder Alkenyloxy mit 1 bzw. 2 bis 6 Kohlenstoffatomen bedeutet, in welchen eine $CH_2$-Gruppe durch Sauerstoff ersetzt sein kann, mit der Massgabe, dass zwei Sauerstoffatome nicht direkt benachbart sind;
- X $-OCF_3$, $-OCHF_2$, $-CH=CF_2$, -CH=CHF, -CH=CHCl, Alkyl, Alkenyl, Alkoxy oder Alkenyloxy mit 1 bzw. 2 bis 3 Kohlenstoffatomen bedeutet;
- $X^1$ Fluor, Chlor, Cyano, $-OCF_3$, $-OCHF_2$ oder $-CH=CF_2$ bedeuten;
- $Z^3$ eine einfache Kovalenzbindung oder $-CH_2CH_2-$ bedeutet; und
- y 0, 1 oder 2 bedeutet; und der Phenylring

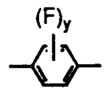

unsubstituiert oder wie folgt substituiert ist

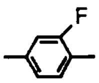 , 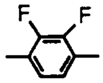 oder 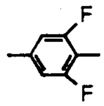 .

Ganz besonders bevorzugte Verbindungen der Formel I-2 sind diejenigen, worin $X^1$ Fluor, Chlor oder Cyano bedeutet.

Weitere, ganz besonders bevorzugte Verbindungen der Formel I sind Verbindungen der Formeln I-3 bis I-10, insbesondere Verbindungen der Formeln I-3 bis I-8, worin X Alkyl oder Alkenyl mit 1 bzw. 2 bis 3 Kohlenstoffatomen bedeutet, wie beispielsweise Methyl, Aethyl, Propyl, Vinyl, 1E-Propenyl, oder aber $-CH=CF_2$, -CH=CHF oder -CH=CHCl darstellt.

Die Herstellung der Verbindungen der Formel I kann in an sich bekannter Weise erfolgen. Sie wird im untenstehenden Schema dargelegt, wobei den in den Formeln I-VI angegebenen Substituenten die obenerwähnte Bedeutung zukommt. Die Halogenierung mit Brom oder Iod einer Verbindung der Formel II zu Verbindungen der Formel III wird vorzugsweise in einem inerten Lösungsmittel (z.B. Tetrahydrofuran, Aether, Dimethoxyäthan usw.) bei tiefer Temperatur (z.B. in einem Bereich von -70° bis -40°) durchgeführt.

Metallkatalysierte Kopplungen von Phenylverbindungen mit Acetylenen, wie sie in der letzten Stufe zur Herstellung von I aus III dargestellt sind, sind grundsätzlich aus der Literatur bekannt, z.B. *C. Pogh and V. Percey, Mol. Cryst. Liq. Cryst.* (1990) 178, 193. Sie können wie bereits erwähnt mit Bromiden und Iodiden, in einigen Fallen aber auch mit Trifluorsulfonaten und Chloriden durchgeführt werden Die Cyclohexylacetylene der Formel VI sind bekannte oder analoge bekannter Verbindungen; solche Verbindungen sind beispielsweise in EP 122 389 beschrieben. Ihre Herstellung ist dem Fachmann bekannt und kann nach üblichen Methoden, beispielsweise nach dem im Schema skizzierten Weg erfolgen Demnach kann ein trans-Cyclohexylcarboxaldehyd der allgemeinen Formel IV einer Wittigreaktion mit Tetrabrommethan und Triphenylphosphin unterworfen werden, und das daraus entstandene 2,2-Dibromvinylcyclohexan V mit Butyllithium zum Acetylen VI umgesetzt werden. Aldehyde der Formel IV oder analoge Aldehyde sind dem Fachmann bekannt und können nach bekannten Methoden hergestellt werden. Viele von ihnen sind als Zwischenprodukte zur Herstellung von Flüssigkristallen beschrieben worden, beispielsweise in *M. Petrzilka und A Germann Mol. Cryst. Liq. Cryst.* (1985) 131, 327.

## Schema

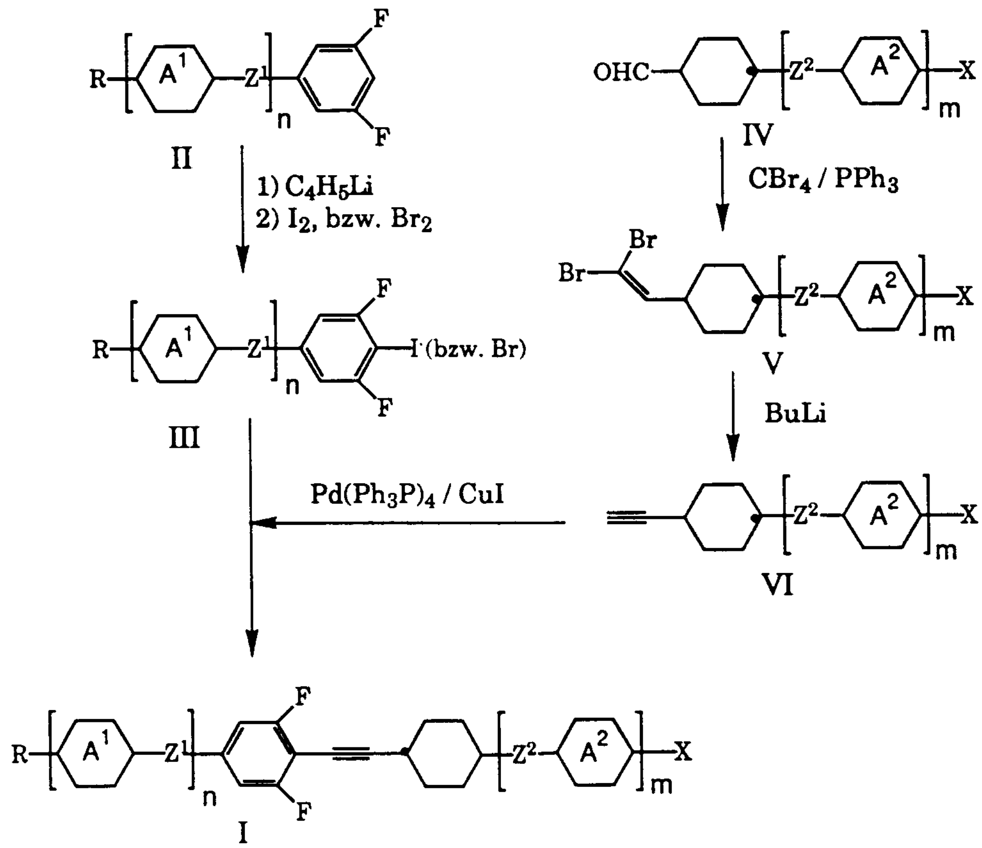


Die Synthese derienigen Verbindungen der Formel II, bei denen n=1 bedeutet, erfolgt in völliger Analogie zu den isomeren 3,4-Difluorphenylverbindungen, die dem Fachmann als Flüssigkristalle geläufig sind. Die Verbindungen der Formel II, bei denen n=0 bedeutet, lassen sich leicht nach bekannten Methoden z.B durch Wittigreaktion und anschliessende katalytische Hydrierung oder durch Verätherung aus kommerziell erhältlichem 3,5-Difluorbenzaldehyd bzw. 3,5-Difluorphenol herstellen.

Die erfindungsgemässen flüssigkristallinen Gemische enthalten mindestens zwei Komponenten, wovon mindestens eine Komponente eine Verbindung der Formel I ist. Eine zweite und gegebenenfalls weitere Komponenten können weitere Verbindungen der Formel I und/oder andere geeignete Flüssigkristallkomponenten sein.

Die Verbindungen der Formel I eignen sich insbesondere für nematische oder, sofern mindestens eine Komponente des Gemisches optisch aktiv ist, auch für cholesterische Gemische. Ein bevorzugter Anwendungsbereich betrifft die Verwendung als Dielektrikum in Flüssigkristallanzeigevorrichtungen mit verdrillt nematischer Flüssigkristallstruktur, wie TN-Zellen, STN-Zellen und TFT-Zellen. Bevorzugte Gemische sind daher diejenigen, welche zusätzlich eine oder mehrere Verbindungen mit positiver dielektrischer Anisotropie enthalten.

Aufgrund der guten Löslichkeit der Verbindungen der Formel I in anderen Flüssigkristallmaterialien und aufgrund ihrer guten Mischbarkeit untereinander kann der Anteil der Verbindungen der Formel I in den erfindungsgemässen Gemischen relativ hoch sein und beispielsweise etwa 1-70 Gew.-% betragen. Im allgemeinen ist ein Anteil von etwa 3-40 Gew.-%, insbesondere 5-30 Gew.-% an Verbindungen der Formel I bevorzugt.

Vorzugsweise enthalten die erfindungsgemässen Gemische neben einer oder mehreren Verbindungen der Formel I, eine oder mehrere Verbindungen aus der Gruppe der Verbindungen der allgemeinen Formeln

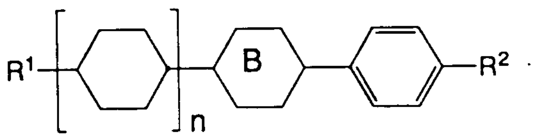

VII

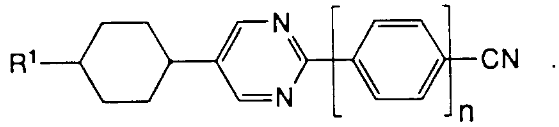

VIII

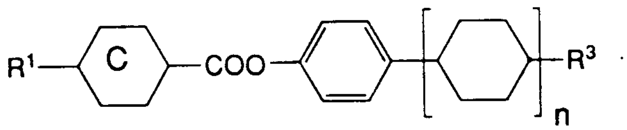

IX

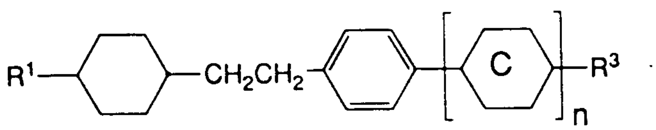

X

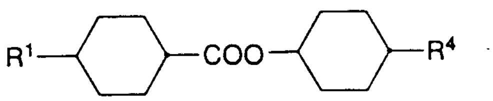

XI

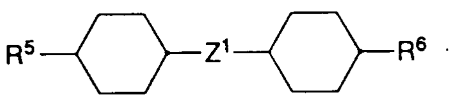

XII

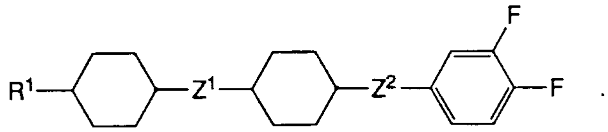

XIII

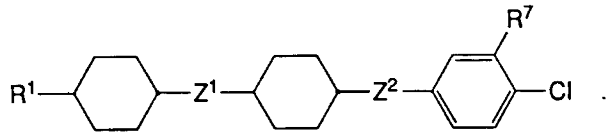

XIV

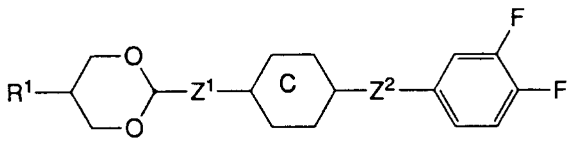

XV

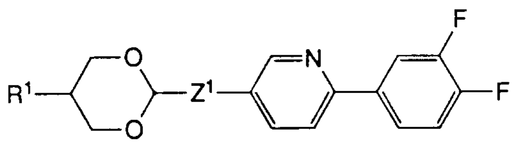

XVI

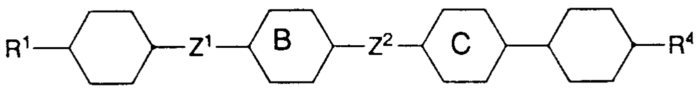

XVII

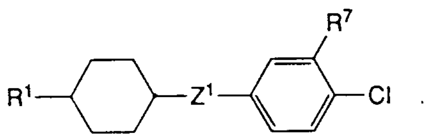

XVIII

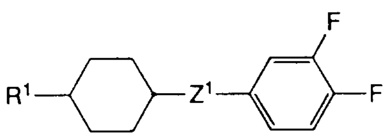

XIX

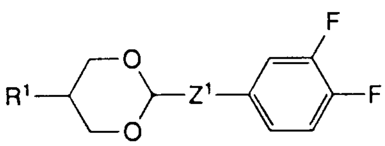

XX

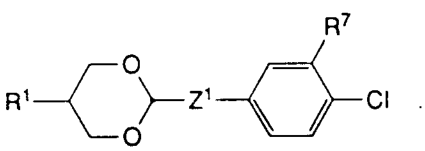

XXI

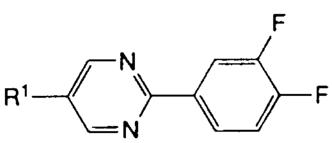

XXII

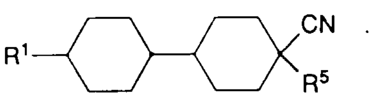

XXIII

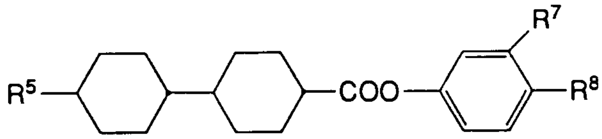

XXIV

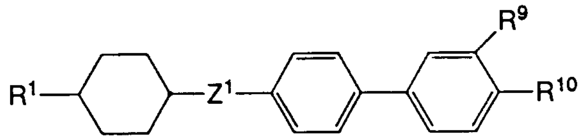

XXV

worin

| | |
|---|---|
| $R^1$,$R^4$ | Alkyl, Alkoxyalkyl, 3E-Alkenyl, 4-Alkenyl oder an gesättigten Ringen auch 1E-Alkenyl bedeuten; |
| n | 0 oder 1 bedeutet; |
| Ring B | 1,4-Phenylen, Pyridin-2,5-diyl, Pyrimidin-2,5-diyl, trans-1,4-Cyclohexylen oder trans-1,3-Dioxan-2,5-diyl bezeichnet; |
| $R^2$ | Cyano, Isothiocyanato, Fluor, Alkyl, 3E-Alkenyl, 4-Alkenyl, Alkoxy, 2E-Alkenyloxy, 3-Alkenyloxy oder 1-Alkinyl darstellt; |
| Ring C | 1,4-Phenylen oder trans-1,4-Cyclohexylen bedeutet; |
| $R^3$ | Alkyl, 3E-Alkenyl, 4-Alkenyl, oder an trans-1,4-Cyclohexylen auch 1E-Alkenyl, oder an 1,4-Phenylen auch Cyano, Isothiocyanato, Alkoxy, 2E-Alkenyloxy oder 3-Alkenyloxy bezeichnet; |
| $R^5$ | Alkyl, 1E-Alkenyl, 3E-Alkenyl oder 4-Alkenyl bedeutet; |
| $R^6$ | Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl darstellt; |
| $Z^1$, $Z^2$ | unabhängig voneinander eine einfache Kovalenzbindung oder $-CH_2CH_2-$ bezeichnen, wobei zwei aromatische Ringe immer durch eine einfache Kovalenzbindung verknüpft sind; |
| $R^7$ | Wasserstoff, Fluor oder Chlor bedeutet; |
| $R^8$ | Cyano, Fluor oder Chlor darstellt; |
| $R^9$ | Wasserstoff oder Fluor bezeichnet; und |
| $R^{10}$ | Fluor oder Chlor darstellt. |

Der obige Ausdruck "gesättigter Ring" umfasst trans-1,4-Cyclohexylen und trans-1,3-Dioxan-2,5-diyl. Die Reste $R^1$ bis $R^6$ besitzen vorzugsweise je 1, bzw. 2 bis 12 Kohlenstoffatome, besonders bevorzugt je 1, bzw. 2 bis 7 Kohlenstoffatome. Geradkettige Reste sind im allgemeinen bevorzugt.

Der Ausdruck "Alkyl" bedeutet in diesem Zusammenhang vorzugsweise geradkettige Reste mit 1 bis 12 Kohlenstoffatomen, vorzugsweise mit 1 bis 7 Kohlenstoffatomen, wie beispielsweise Methyl, Aethyl, Propyl, Butyl, Pentyl, Hexyl, Heptyl und dergleichen.

Der Ausdruck "Alkyloxyalkyl" bedeutet in diesem Zusammenhang vorzugsweise geradkettige Reste mit 1 bis 12 Kohlenstoffatomen, besonders bevorzugt mit 1 bis 7 Kohlenstoffatomen, wie beispielsweise Methoxymethyl, Aethoxymethyl, Propyloxyethyl, Butyloxymethyl, Methoxypropyl und dergleichen.

Der Ausdruck "Alkoxy" bedeutet in diesem Zusammenhang vorzugsweise geradkettige Reste mit 1 bis 12 Kohlenstoffatomen, besonders bevorzugt mit 1 bis 7 Kohlenstoffatomen, wie beispielsweise Methoxy, Aethoxy, Propyloxy, Butyloxy, Pentyloxy, Hexyloxy, Heptyloxy und dergleichen.

Der Ausdruck "1E-Alkenyl" bedeutet in diesem Zusammenhang vorzugsweise geradkettige Alkenylreste mit 2 bis 12, besonders bevorzugt mit 2 bis 7 Kohlenstoffatomen, in denen die Doppelbindung sich in 1-Stellung befindet, wie beispielsweise Vinyl, 1E-Propenyl, 1E-Butenyl, 1E-Pentenyl, 1E-Hexenyl, 1E-Heptenyl und dergleichen.

Der Ausdruck "3E-Alkenyl" bedeutet in diesem Zusammenhang vorzugsweise geradkettige Alkenylreste mit 4 bis 12, besonders bevorzugt mit 4 bis 7 Kohlenstoffatomen, in denen die Doppelbindung sich in 3-Stellung befindet, wie beispielsweise 3-Butenyl, 3E-Pentenyl, 3E-Hexenyl, 3E-Heptenyl und dergleichen.

Der Ausdruck "4-Alkenyl"" bedeutet in diesem Zusammenhang vorzugsweise geradkettige Alkenylreste mit 5 bis 12 Kohlenstoffatomen, in denen die Doppelbindung sich in 4-Stellung befindet, wie beispielsweise 4-Pentenyl, 4-Hexenyl, 4-Heptenyl und dergleichen.

Der Ausdruck "2E- bzw. 3-Alkenyloxy" bedeutet in diesem Zusammenhang vorzugsweise geradkettige Alkenyloxyreste mit 3 bzw. 4 bis 12 Kohlenstoffatomen, besonders bevorzugt mit 3 bzw. 4 bis 7 Kohlenstoffatomen, in denen die Doppelbindung sich in 2- bzw. 3-Stellung befindet und E die bevorzugte Konfiguration angibt, wie beispielsweise Allyloxy, 2E-Butenyloxy, 2E-Pentenyloxy, 2E-Hexenyloxy, 2E-Heptenyloxy, 3-Butenyloxy, 3-Pentenyloxy, 3-Hexenyloxy, 3-Heptenyloxy, 4-Pentenyloxy, 5-Hexenyloxy, 6-Heptenyloxy und dergleichen.

Der Ausdruck "1-Alkinyl" bedeutet in diesem Zusammenhang vorzugsweise geradkettige Alkinylreste mit 2 bis 12, besonders bevorzugt mit 2 bis 7 Kohlenstoffatomen, in denen die Dreifachbindung sich in 1-Stellung befindet, wie beispielsweise Aethinyl, 1-Propinyl, 1-Butinyl, 1-Pentinyl und dergleichen.

Die Herstellung der flüssigkristallinen Gemische und der elektrooptischen Vorrichtungen kann in an sich bekannter Weise erfolgen.

Die Erfindung wird durch die folgenden Beispiele weiter veranschaulicht. In den Beispielen bedeutet C eine kristalline, N eine nematische, S eine smektische und I die isotrope Phase. $V_{10}$ bezeichnet die Spannung für 10% Transmission; $t_{on}$ und $t_{off}$ bezeichnen die Einschaltzeit bzw. die Ausschaltzeit, und $\Delta n$ bezeichnet die optische Anisotropie.

Beispiel 1

a) Zu einer Lösung von 2,14g 1-(trans-4-Propylcyclohexyl)-3,5-difluorbenzol in 20 ml trockenem Tetrahydrofuran bei -70°C werden 5,9 ml einer 1,6 N Butyllithiumlösung in Hexan wahrend 20 Min. getropft und während 1 Std. bei -70°C reagieren gelassen. Dann wird bei -60° eine Lösung von 2,4g Iod in 10 ml trockenem Tetrahydrofuran innert 10 Min. zugetropft und während weiteren 30 Min. allmählich auf Raumtemperatur erwärmt. Die entstandene gelbe Lösung wird danach mit 10 ml Wasser und dann mit 10 ml einer 10proz. wässrigen Natriumbicarbonatlösung versetzt und mit Aether extrahiert. Die Aetherlösung wird mit gesättigter Kochsalzlösung und mehrmals mit Wasser gewaschen, über Magnesiumsulfat getrocknet, filtriert und eingeengt. Chromatographie des Rückstandes über 150 g Kieselgel mit Hexan ergab 3,27 g 1-(trans-4-Propylcyclohexyl)-3,5-difluor-4-iodbenzol als farblose Flüssigkeit.

b) Ein Gemisch von 0,8g 1-(trans-4-Propylcyclohexyl)-3,5-difluor-4-iodbenzol, 0,397 g trans-1-Aethinyl-4-propylcyclohexan, 0,023 g Tetrakis-(triphenylphosphin)palladium(0) und 0,009g Kupfer(I)iodid in 20 ml Triäthylamin wird während 17 Std. bei 105°C gerührt. Danach wird abgekühlt, zwischen Aether und Wasser verteilt, die vereinigten organischen Phasen über Magnesiumsulfat getrocknet, abfiltriert und am Rotationsverdampfer eingedampft. Chromatographie an 140 g Kieselgel mit Hexan und dreimalige Kristallisation aus Methanol, Hexan und wieder Methanol ergibt 0,376 g 1-(trans-4-Propylcyclohexyläthinyl)-2,6-difluor-4-(trans-4-propylcyclohexyl)benzol, Smp. (C/N) 120,2°C, Klp. (N/I) 171,4°C.

Auf analoge Weise können die folgenden Verbindungen hergestellt werden:

1-(trans-4-Aethylcyclohexyläthinyl)-2,6-difluor-4-propylbenzol, Smp. (C/I) 1,2°C, Klp. (N/I) -43°C (virt.);
1-(trans-4-Aethylcyclohexyläthinyl)-2,6-difluor-4-butylbenzol;
1-(trans-4-Aethylcyclohexyläthinyl)-2,6-difluor-4-pentylbenzol;
1-(trans-4-Vinylcyclohexyläthinyl)-2,6-difluor-4-propylbenzol;
1-(trans-4-Propylcyclohexyläthinyl)-2,6-difluor-4-propylbenzol;
1-(trans-4-Propylcyclohexyläthinyl)-2,6-difluor-4-butylbenzol;
1-(trans-4-Propylcyclohexyläthinyl)-2,6-difluor-4-pentylbenzol;
1-(trans-4-Butylcyclohexyläthinyl)-2,6-difluor-4-propylbenzol;
1-(trans-4-Methoxycyclohexyläthinyl)-2,6-difluor-4-propylbenzol;
1-(trans-4-Aethoxycyclohexyläthinyl)-2,6-difluor-4-propylbenzol, Smp. Smp. (C/N) 41,8°C, Klp. (N/I) -80°C (virt.);
1-(trans-4-Difluormethoxycyclohexyläthinyl)-2,6-difluor-4-propylbenzol;
1-(trans-4-Trifluormethoxycyclohexyläthinyl)-2,6-difluor-4-propylbenzol;
1-[trans-4-(2-Fluorvinyl)cyclohexyläthinyl]-2,6-difluor-4-propylbenzol;
1-[trans-4-(2,2-Difluorvinyl)cyclohexyläthinyl]-2,6-difluor-4-propylbenzol, Klp. (N/I) -35°C (virt.);
1-[trans-4-(2-Fluorvinyl)cyclohexyläthinyl]-2,6-difluor-4-butylbenzol;
1-[trans-4-(2,2-Difluorvinyl)cyclohexyläthinyl]-2,6-difluor-4-butylbenzol;
1-[trans-4-(2-Fluorvinyl)cyclohexyläthinyl]-2,6-difluor-4-(3-butenyl)-benzol;
1-[trans-4-(2,2-Difluorvinyl)cyclohexyläthinyl]-2,6-difluor-4-(3-butenyl)benzol;
1-[trans-4-(2,2-Difluorvinyl)cyclohexyläthinyl]-2,6-difluor-4-pentylbenzol;
1-[trans-4-(2-Fluorvinyl)cyclohexyläthinyl]-2,6-difluor-4-pentylbenzol;
1-[trans-4-(2,2-Difluorvinyl)cyclohexyläthinyl]-2,6-difluor-4-(4-pentenyl)benzol;
1-[trans-4-(2-Fluorvinyl)cyclohexyläthinyl]-2,6-difluor-4-(4-pentenyl)benzol;
1-[trans-4-(2-E-Chlorvinyl)cyclohexyläthinyl]-2,6-difluor-4-propylbenzol;
1-[trans-4-(2-E-Chlorvinyl)cyclohexyläthinyl]-2,6-difluor-4-butylbenzol;
1-[trans-4-(2-E-Chlorvinyl)cyclohexyläthinyl]-2,6-difluor-4-(3-butenyl)benzol;
1-[trans-4-(2-E-Chlorvinyl)cyclohexyläthinyl]-2,6-difluor-4-pentylbenzol;
1-[trans-4-(2-E-Chlorvinyl)cyclohexyläthinyl]-2,6-difluor-4-(4-pentenyl)benzol;
1-(trans-4-Aethylcyclohexyläthinyl)-2,6-difluor-4-(trans-4-propylcyclohexyl)benzol, Smp. (C/N) 86,3°C, Klp. (N/I) 152,2°C;

1-(trans-4-Aethylcyclohexyläthinyl)-2,6-difluor-4-(trans-4-butylcyclohexyl)benzol;
1-(trans-4-Aethylcyclohexyläthinyl)-2,6-difluor-4-(trans-4-pentylcyclohexyl)benzol;
1-(trans-4-Propylcyclohexyläthinyl)-2,6-difluor-4-(trans-4-butylcyclohexyl)benzol;
1-(trans-4-Propylcyclohexyläthinyl)-2,6-difluor-4-(trans-4-pentylcyclohexyl)benzol;
1-(trans-4-Butylcyclohexyläthinyl)-2,6-difluor-4-(trans-4-propylcyclohexyl)benzol, Smp. (C/N) 93,6 °C, Klp. (N/I) 167 °C;
1-(trans-4-Butylcyclohexyläthinyl)-2,6-difluor-4-(trans-4-butylcyclohexyl)benzol;
1-(trans-4-Butylcyclohexyläthinyl)-2,6-difluor-4-(trans-4-pentylcyclohexyl)benzol;
1-(trans-4-Vinylcyclohexyläthinyl)-2,6-difluor-4-(trans-4-propylcyclohexyl)benzol;
1-(trans-4-Difluormethoxycyclohexyläthinyl)-2,6-difluor-4-(trans-4-propylcyclohexyl)benzol;
1-(trans-4-Aethylcyclohexyläthinyl)-2,6-difluor-4-(trans-4-vinylcyclohexyl)benzol;
1-(trans-4-Aethylcyclohexyläthinyl)-2,6-difluor-4-[trans-4-(1-E-propenyl)cyclohexyl]benzol;
1-(trans-4-Aethylcyclohexyläthinyl)-2,6-difluor-4-[trans-4-(3-butenyl)cyclohexyl]benzol;
1-(trans-4-Aethylcyclohexyläthlnyl)-2,6-difluor-4-[trans-4-(4-pentenyl)cyclohexyl]benzol;
1-[trans-4-(2-Fluorvinyl)cyclohexyläthinyl]-2,6-difluor-4-(trans-4-äthylcyclohexyl)benzol;
1-[trans-4-(2,2-Difluorvinyl)cyclohexyläthinyl]-2,6-difluor-4-(trans-4-äthylcyclohexyl)benzol;
1-[trans-4-(2-Fluorvinyl)cyclohexyläthinyl]-2,6-difluor-4-(trans-4-propylcyclohexyl)benzol;
1-[trans-4-(2,2-Difluorvinyl)cyclohexyläthinyl]-2,6-difluor-4-(trans-4-propylcyclohexyl)benzol, Smp. (C/N) 53,1°, (N/C <35°C), Klp. (N/I) 169,9°C;
1-[trans-4-(2-Fluorvinyl)cyclohexyläthinyl]-2,6-difluor-4-(trans-4-pentylcyclohexyl)benzol;
1-[trans-4-(2,2-Difluorvinyl)cyclohexyläthinyl]-2,6-difluor-4-(trans-4-pentylcyclohexyl)benzol;
1-[trans-4-(2-E-Chlorvinyl)cyclohexyläthinyl]-2,6-difluor-4-(trans-4-propylcyclohexyl)benzol;
1-[trans-4-(2-E-Chlorvinyl)cyclohexyläthinyl]-2,6-difluor-4-(trans-4-pentylcyclohexyl)benzol;
1-(trans-4-Methoxycyclohexyläthinyl])-2,6-difluor-4-(trans-4-propylcyclohexyl)benzol;
1-(trans-4-Aethoxycyclohexyläthinyl])-2,6-difluor-4-(trans-4-propylcyclohexyl)benzol, Smp. (C/N) 93,3°C, Klp. (N/I) 112,1°C;
1-(trans-4-Difluormethoxycyclohexyläthinyl)-2,6-difluor-4-(trans-4-propylcyclohexyl)benzol;
1-(trans-4-Trifluormethoxycyclohexyläthinyl])-2,6-difluor-4-(trans-4-propylcyclohexyl)benzol;
1-(trans-4-Cyanocyclohexyläthinyl)-2,6-difluor-4-(trans-4-propylcyclohexyl)benzol;
1-(trans-4-Aethylcyclohexyläthinyl)-2,6-difluor-4-(trans-5-propyl-1,3-dioxan-2-yl)benzol;
1-(trans-4-Aethylcyclohexyläthinyl)-2,6-difluor-4-(trans-5-pentyl-1,3-dioxan-2-yl)benzol;
1-(trans-4-Aethylcyclohexyläthinyl)-2,6-difluor-4-[trans-5-(1-E-propenyl)-1,3-dioxan-2-yl]benzol;
1-(trans-4-Propylcyclohexyläthinyl)-2,6-difluor-4-[trans-5-(4-pentenyl)-1,3-dioxan-2-yl]benzol;
1-[trans-4-(2-Fluorvinyl)cyclohexyläthinyl]-2,6-difluor-4-(trans-5-propyl-1,3-dioxan-2-yl)benzol;
1-[trans-4-(2,2-Difluorvinyl)cyclohexyläthinyl]-2,6-difluor-4-(trans-5-propyl-1,3-dioxan-2-yl)benzol;
1-[trans-4-1-E-Chlorvinyl)cyclohexyläthinyl]-2,6-difluor-4-(trans-5-propyl-1,3-dioxan-2-yl)benzol;
1-(trans-4-Aethylcyclohexyläthinyl)-2,6-difluor-4-(4-propylphenyl)benzol;
1-(trans-4-Aethylcyclohexyläthinyl)-2,6-difluor-4-(4-butylphenyl)benzol;
1-(trans-4-Propylcyclohexyläthinyl)-2,6-difluor-4-(4-butylphenyl)benzol;
1-(trans-4-Propylcyclohexyläthinyl)-2,6-difluor-4-(4-pentylphenyl)benzol;
1-(trans-4-Propylcyclohexyläthinyl)-2,6-difluor-4-[4-(4-pentenyl)phenyl]benzol;
1-(trans-4-Propylcyclohexyläthinyl)-2,6-difluor-4-(4-propyloxyphenyl)benzol;
1-[trans-4-(2-Fluorvinyl)cyclohexyläthinyl]-2,6-difluor-4-(4-propylphenyl)benzol;
1-[trans-4-(2,2-Difluorvinyl)cyclohexyläthinyl]-2,6-difluor-4-(4-propylphenyl)benzol;
1-[trans-4-(2-E-Chlorvinyl)cyclohexyläthinyl]-2,6-difluor-4-(4-propylphenyl)benzol;
1-[trans-4-Aethylcyclohexyläthinyl]-2,6-difluor-4-(5-propylpyrimidin-2-yl)benzol;
1-[trans-4-Propylcyclohexyläthinyl]-2,6-difluor-4-(5-butylpyrimidin-2-yl)benzol;
1-[trans-4-Propylcyclohexyläthinyl]-2,6-difluor-4-(5-pentylpyrimidin-2-yl)benzol;
1-[trans-4-Butylcyclohexyläthinyl]-2,6-difluor-4-(5-propylpyrimidin-2-yl)benzol;
1-[trans-4-(2-Fluorvinyl)cyclohexyläthinyl]-2,6-difluor-4-(5-pentylpyrimidin-2-yl)benzol;
1-[trans-4-(2,2-Difluorvinyl)cyclohexyläthinyl]-2,6-difluor-4-(5-pentylpyrimidin-2-yl)benzol;
1-[trans-4-(2-E-Chlorvinyl)cyclohexyläthinyl]-2,6-difluor-4-(5-pentylpyrimidin-2-yl)benzol;
1-[trans-4-Aethylcyclohexyläthinyl]-2,6-difluor-4-(5-propylpyridin-2-yl)benzol;
1-[trans-4-Butylcyclohexyläthinyl]-2,6-difluor-4-(5-propylpyridin-2-yl)benzol;
1-[trans-4-(2-Fluorvinyl)cyclohexyläthinyl]-2,6-difluor-4-(5-propylpyridin-2-yl)benzol;
1-[trans-4-(2,2-Difluorvinyl)cyclohexyläthinyl]-2,6-difluor-4-(5-propylpyridin-2-yl)benzol;
1-[trans-4-(2-E-Chlorvinyl)cyclohexyläthlnyl]-2,6-difluor-4-(5-pentylpyridin-2-yl)benzol;
1-(trans-4-Aethylcyclohexyläthinyl)-2,6-difluor-4-[2-(trans-4-propylcyclohexyl)äthyl]benzol;
1-(trans-4-Aethylcyclohexyläthinyl)-2,6-difluor-4-[2-(trans-4-butylcyclohexyl)äthyl]benzol;

1-(trans-4-Aethylcyclohexyläthinyl)-2,6-difluor-4-[2-(trans-4-pentylcyclohexyl)äthyl]benzol;
1-(trans-4-Aethylcyclohexyläthinyl)-2,6-difluor-4-[2-(trans-4-(3-butenyl)cyclohexyl)äthyl]benzol;
1-(trans-4-Vinylcyclohexyläthinyl)-2,6-difluor-4-[2-(trans-4-propylcyclohexyl)äthyl]benzol;
1-(trans-4-Propylcyclohexyläthinyl)-2,6-difluor-4-[2-(trans-4-propylcyclohexyl)äthyl]benzol;
1-[trans-4-(2-Fluorvinyl)cyclohexyläthinyl]-2,6-difluor-4-[2-(trans-4-propylcyclohexyl)äthyl]benzol;
1-[trans-4-(2,2-Difluorvinyl)cyclohexyläthinyl]-2,6-difluor-4-[2-(trans-4-propylcyclohexyl)äthyl]benzol;
1-[trans-4-(2-E-Chlorvinyl)cyclohexyläthinyl]-2,6-difluor-4-[2-(trans-4-butylcyclohexyl)äthyl]benzol;
1-(trans-4-Aethylcyclohexyläthinyl)-2,6-difluor-4-[2-(trans-5-propyl-1,3-dioxan-2-yl)äthyl]benzol;
1-(trans-4-Aethylcyclohexyläthinyl)-2,6-difluor-4-[2-(trans-5-butyl-1,3-dioxan-2-yl)äthyl]benzol;
1-(trans-4-Aethylcyclohexyläthinyl)-2,6-difluor-4-[2-(trans-5-(3-butenyl)-1,3-dioxan-2-yl)äthyl]benzol;
1-(trans-4-Aethylcyclohexyläthinyl)-2,6-difluor-4-[2-(trans-5-pentyl-1,3-dioxan-2-yl)äthyl]benzol;
1-(trans-4-Vinylcyclohexyläthinyl)-2,6-difluor-4-[2-(trans-5-propyl-1,3-dioxan-2-yl)äthyl]benzol;
1-(trans-4-Propylcyclohexyläthinyl)-2,6-difluor-4-[2-(trans-5-propyl-1,3-dioxan-2-yl)äthyl]benzol;
1-[trans-4-(2-Fluorvinyl)cyclohexyläthinyl]-2,6-difluor-4-[2-(trans-5-propyl-1,3-dioxan-2-yl)äthyl]benzol;
1-[trans-4-(2,2-Difluorvinyl)cyclohexyläthinyl]-2,6-difluor-4-[2-(trans-5-propyl-1,3-dioxan-2-yl)äthyl]benzol;
1-[trans-4-(2-E-Chlorvinyl)cyclohexyläthinyl]-2,6-difluor-4-[2-(trans-5-butyl-1,3-dioxan-2-yl)äthyl]benzol;

Beispiel 2

Ein Gemisch von 0,65 g 1-Propyl-3,5-difluor-4-iodbenzol (hergestellt gemäss Beispiel 1a), 0,56 g trans-4-[trans-4-(3-Butenyl)cyclohexyl]cyclohexylacetylen, 0,053 g Tetrakis(triphenylphosphin)palladium(0), 0,009 g Kupfer(I)iodid und 6 ml Triethylamin wurde während 17 Std. auf 90°C erhitzt. Das Reaktionsgemisch wurde abgekühlt, zwischen Wasser und Aether verteilt, die organische Phase wurde abgetrennt und die wässrige Phase mahrmals mit Aether extrahiert. Die vereinigten organischen Phasen wurden über Magnesiumsulfat getrocknet, filtriert und eingedampft. Chromatographie des Rückstandes an Kieselgel mit Hexan und zweimalige Kristallisation, zuerst aus Hexan und danach aus Isopropanol ergab 640 mg 1-{trans-4-[trans-4-(3-Butenyl)cyclohexyl]cyclohexyläthinyl}-2,6-difluor-4-propylbenzol, Smp. (C/N) 48,5°C, Klp. (N/I) 189,8°C.

Auf analoge Weise können die folgenden Verbindungen hergestellt werden:

1-[trans-4-(trans-4-Aethylcyclohexyl)cyclohexyläthinyl]-2,6-difluor-4-propylbenzol, Smp. (C/N) 41,6°C, (N/C) <35°C), Klp. (N/I) 163,1°C;
1-[trans-4-(trans-4-Aethylcyclohexyl)cyclohexyläthinyl]-2,6-difluor-4-butylbenzol;
1-[trans-4-(trans-4-Propylcyclohexyl)cyclohexyläthinyl]-2,6-difluor-4-pentylbenzol;
1-[trans-4-(trans-4-Aethylcyclohexyl)cyclohexyläthinyl]-2,6-difluor-4-(4-pentenyl)benzol;
1-[trans-4-(trans-4-Vinylcyclohexyl)cyclohexyläthinyl]-2,6-difluor-4-propylbenzol, Smp. (C/N) 48,8°C, Klp. (N/I) 181,1°C;
1-{trans-4-[trans-4-(3-Butenyl)cyclohexyl]cyclohexyläthinyl}-2,6-difluor-4-propylbenzol, Smp. (C/N) 48°C, Klp. (N/I) 189,8°C;
1-{trans-4-[trans-4-(4-Pentenyl)cyclohexyl]cyclohexyläthinyl}-2,6-difluor-4-propylbenzol, Smp. (C/N) 31,7°C, Klp. (N/I) 182°C;
1-[trans-4-(trans-4-Vinylcyclohexyl)cyclohexyläthinyl]-2,6-difluor-4-äthoxybenzol, Smp. (C/N) 99°C, Klp. (N/I) 219,6°C;
1-{trans-4-[trans-4-(2-Fluorvinyl)cyclohexyl]cyclohexyläthinyl}-2,6-difluor-4-propylbenzol;
1-{trans-4-[trans-4-(2,2-Difluorvinyl)cyclohexyl]cyclohexyläthinyl)}-2,6-difluor-4-propylbenzol, Smp. (C/N) 51,5°C, Klp. (N/I) 192,6°C;
1-{trans-4-[trans-4-(2-E-Chlorvinyl)cyclohexyl]cyclohexyläthinyl}-2,6-difluor-4-propylbenzol, Smp. (C/N) 80,2°C, Klp. (N/I) 245,5°C;
1-{trans-4-[trans-4-(2-E-Chlorvinyl)cyclohexyl]cyclohexyläthinyl}-2,6-difluor-4-äthoxybenzol, Smp. (C/N) 102,1°C, Klp. (N/I) 287,5°C;
1-[trans-4-(trans-4-Methoxycyclohexyl]cyclohexyläthinyl]-2,6-difluor-4-propylbenzol;
1-[trans-4-(trans-4-Methoxymethylcyclohexyl]cyclohexyläthinyl]-2,6-difluor-4-propylbenzol;
1-[trans-4-(trans-4-Allyloxycyclohexyl]cyclohexyläthinyl]-2,6-difluor-4-propylbenzol;
1-[trans-4-(trans-4-Aethylcyclohexyl]cyclohexyläthinyl]-2,6-difluor-4-butyloxybenzol;
1-{trans-4-[2-(trans-4-Propylcyclohexyl)äthyl]cyclohexyläthinyl}-2,6-difluor-4-propylbenzol, Smp. (C/N) 64°C, Klp. (N/I) 153,8°C;
1-{trans-4-[2-(trans-4-Propylcyclohexyl)äthyl]cyclohexyläthinyl}-2,6-difluor-4-propylbenzol;
1-{trans-4-[2-(trans-4-Aethylcyclohexyl)äthyl]cyclohexyläthinyl}-2,6-difluor-4-pentylbenzol;
1-{trans-4-[2-(trans-4-Vinylcyclohexyl)äthyl]cyclohexyläthinyl}-2,6-difluor-4-propylbenzol;
1-{trans-4-[2-(trans-4-(2-Fluorvinyl)cyclohexyl)äthyl]cyclohexyläthinyl}-2,6-difluor-propylbenzol;

1-{trans-4-[2-(trans-4-(2,2-Difluorvinyl)cyclohexyl)äthyl]cyclohexyläthinyl}-2,6-difluor-4-propylbenzol;
1-[trans-4-(4-Ethylphenyl)cyclohexyläthinyl]-2,6-difluor-4-propylbenzol;
1-[trans-4-(4-Trifluormethylphenyl)cyclohexyläthinyl]-2,6-difluor-4-propylbenzol;
1-[trans-4-(4-Methoxyphenyl)cyclohexyläthinyl]-2,6-difluor-4-propylbenzol;
1-[trans-4-(4-Difluormethoxyphenyl)cyclohexyläthinyl]-2,6-difluor-4-propylbenzol;
1-[trans-4-(4-Trifluormethoxyphenyl)cyclohexyläthinyl]-2,6-difluor-4-propylbenzol;
1-[trans-4-(4-Cyanophenyl)cyclohexyläthinyl]-2,6-difluor-4-propylbenzol;
1-[trans-4-(4-Fluorphenyl)cyclohexyläthinyl]-2,6-difluor-4-propylbenzol;
1-[trans-4-(4-Chlorphenyl)cyclohexyläthinyl]-2,6-difluor-4-propylbenzol;
1-[trans-4-(3,4-Difluorphenyl)cyclohexyläthinyl]-2,6-difluor-4-äthylbenzol, Smp (C/I) 71,7°C, Klp. (N/I) 61,2°C;
1-[trans-4-(3,4-Difluorphenyl)cyclohexyläthinyl]-2,6-difluor-4-propylbenzol, Smp. (C/N) 54,4°C, Klp. (N/I) 70,2°C;
1-[trans-4-(3,4-Difluorphenyl)cyclohexyläthinyl]-2,6-difluor-4-butylbenzol, Smp (C/I) 47,5°C, Klp. (N/I) 62°C;
1-[trans-4-(3-Fluor-4-Chlorphenyl)cyclohexyläthinyl]-2,6-difluor-4-propylbenzol, Smp (C/I) 65°C, Klp. (N/I) 114,3°C;
1-[trans-4-(3-Fluor-4-Cyanophenyl)cyclohexyläthinyl]-2,6-difluor-4-propylbenzol;
1-[trans-4-(3-Fluor-4-Difluormethoxyphenyl)cyclohexyläthinyl]-2,6-difluor-4-propylbenzol;
1-[trans-4-(3,4,5-Trifluorphenyl)cyclohexyläthinyl]-2,6-difluor-4-äthylbenzol, Smp (C/I) 77,1°C, Klp. (N/I) < 45°C
1-[trans-4-(3,4,5-Trifluorphenyl)cyclohexyläthinyl]-2,6-difluor-4-propylbenzol, Smp (C/I) 63,7°C, Klp. (N/I) < 20°C;
1-[trans-4-(3,4,5-Trifluorphenyl)cyclohexyläthinyl]-2,6-difluor-4-butylbenzol, Smp (C/I) 77,1°C, Klp. (N/I) < 25°C
1-[trans-4-(3,5-Difluor-4-chlorphenyl)cyclohexyläthinyl]-2,6-difluor-4-propylbenzol, Smp (C/I) 83,5°C, Klp. (N/I) 80°C
1-[4-trans-4-(3,5-Difluor-4-trifluormethylphenyl)cyclohexyläthinyl]-2,6-difluor-4-propylbenzol, Smp. (C/I) 123,4°C, Klp. -15° (virt.);
1-[trans-4-(2,3,4-Trifluorphenyl)cyclohexyläthinyl]-2,6-difluor-4-propylbenzol;
1-{trans-4-[2-(4-Methylphenyl)äthyl]cyclohexyläthinyl}-2,6-difluor-4-propylbenzol;
1-{trans-4-[2-(4-Ethylphenyl)äthyl]cyclohexyläthinyl}-2,6-difluor-4-propylbenzol;
1-{trans-4-[2-(4-Trifluormethylphenyl)äthyl]cyclohexyläthinyl}-2,6-difluor-4-propylbenzol;
1-{trans-4-[2-(4-Difluormethoxyphenyl)äthyl]cyclohexyläthinyl}-2,6-difluor-4-propylbenzol;
1-{trans-4-[2-(3-Fluor-4-difluormethoxyphenyl)äthyl]cyclohexyläthinyl}-2,6-difluor-4-propylbenzol;
1-{trans-4-[2-(3,4-Difluorphenyl)äthyl]cyclohexyläthinyl}-2,6-difluor-4-propylbenzol, Smp. (C/N) 62,5°C, (N/C) < 28°C, Klp. (N/I) 61,1°C;
1-{trans-4-[2-(3-Fluor-4-chlorphenyl)äthyl]cyclohexyläthinyl}-2,6-difluor-4-propylbenzol;
1-{trans-4-[2-(3,4,5-Trifluorphenyl)äthyl]cyclohexyläthinyl}-2,6-difluor-4-propylbenzol.

Beispiel 3

Zur Untersuchung der Eigenschaften der Verbindungen der Formel I in Gemischen wurden binäre Mischungen (BM) mit 4-(trans-4-Pentylcyclohexyl)benzonitril hergestellt. Die Schwellenspannung und die Ansprechzeiten wurden in einer TN-Zelle (low bias tilt) mit 8 mm Plat-tenabstand bei 22°C gemessen; als Betriebsspannung wurde der 2,5-fache Wert der Schwellenspannung ($V_{10}$) gewählt. Die entsprechenden Daten für 4-(trans-4-Pentylcyclohexyl)benzonitril betragen: Klp. (N/I) = 54.6°C, $V_{10}$ = 1,62 V, $t_{on}$ = 22 ms, $t_{off}$ = 42 ms, $\Delta n$ = 0,120.

BM-1

| | |
|---|---|
| 90 Gew.% | 4-(trans-4-Pentyl-cyclohexyl)benzonitril |
| 10 Gew.% | 1-(trans-4-Propylcyclohexyläthinyl)-2,6-difluor-4-(trans-4-propylcyclohexyl)benzol |
| Klp. (N/I): | 60,7°C, $V_{10}$ = 1,62 V, $t_{on}$ = 27 ms, $t_{off}$ = 45 ms, $\Delta n$ = 0,124 |

BM-2

| | |
|---|---|
| 80 Gew.% | 4-(trans-4-Pentyl-cyclohexyl)benzonitril |
| 20 Gew.% | 1-(trans-4-Propylcyclohexyläthinyl)-2,6-difluor-4-(trans-4-propylcyclohexyl)benzol |
| Klp. (N/I): | 67,6°C, $V_{10}$ = 1,68 V, $t_{on}$ = 30 ms, $t_{off}$ = 47 ms, $\Delta n$ = 0,127 |

BM-3

| | |
|---|---|
| 90 Gew.% | 4-(trans-4-Pentyl-cyclohexyl)benzonitril |
| 10 Gew.% | 1-(trans-4-Butylcyclohexyläthinyl)-2,6-difluor-4-(trans-4-propylcyclohexyl)benzol |
| Klp. (N/I): | 60,4°C, $V_{10}$ = 1,61 V, $t_{on}$ = 28 ms, $t_{off}$ = 45 ms, $\Delta n$ = 0,124 |

BM-4

| | |
|---|---|
| 80 Gew.% | 4-(trans-4-Pentyl-cyclohexyl)benzonitril |
| 20 Gew.% | 1-(trans-4-Butylcyclohexyläthinyl)-2,6-difluor-4-(trans-4-propylcyclohexyl)benzol |
| Klp. (N/I): | 67,3°C, $V_{10}$ = 1,68 V, $t_{on}$ = 32 ms, $t_{off}$ = 51 ms, $\Delta n$ = 0,125 |

BM-5

| | |
|---|---|
| 90 Gew.% | 4-(trans-4-Pentyl-cyclohexyl)benzonitril |
| 10 Gew.% | 1-[trans-4-(trans-4-Aethylcyclohexyl)cyclohexyläthinyl]-2,6-difluor-4-propylbenzol |
| Klp. (N/I): | 60,8°C, $V_{10}$ = 1,68 V, $t_{on}$ = 26 ms, $t_{off}$ = 45 ms, $\Delta n$ = 0,125 |

BM-6

| | |
|---|---|
| 80 Gew.% | 4-(trans-4-Pentyl-cyclohexyl)benzonitril |
| 20 Gew.% | 1-[trans-4-(trans-4-Aethylcyclohexyl)cyclohexyläthinyl]-2,6-difluor-4-propylbenzol |
| Klp. (N/I): | 67,3°C, $V_{10}$ = 1,69 V, $t_{on}$ = 29 ms, $t_{off}$ = 48 ms, $\Delta n$ = 0,126 |

BM-7

| | |
|---|---|
| 90 Gew.% | 4-(trans-4-Pentyl-cyclohexyl)benzonitril |
| 10 Gew.% | 1-{trans-4-[trans-4-(3-Butenyl)cyclohexyl]cyclohexyläthinyl}-2,6-difluor-4-propylbenzol |
| Klp. (N/I): | 63,0°C, $V_{10}$ = 1,67 V, $t_{on}$ = 27 ms, $t_{off}$ = 45 ms, $\Delta n$ = 0,126 |

BM-8

| | |
|---|---|
| 80 Gew.% | 4-(trans-4-Pentyl-cyclohexyl)benzonitril |
| 20 Gew.% | 1-{trans-4-[trans-4-(3-Butenyl)cyclohexyl]cyclohexyläthinyl}-2,6-difluor-4-propylbenzol |
| Klp. (N/I): | 72,3°C, $V_{10}$ = 1,79 V, $t_{on}$ = 27 ms, $t_{off}$ = 46 ms, $\Delta n$ = 0,130 |

BM-9

| | |
|---|---|
| 90 Gew.% | 4-(trans-4-Pentyl-cyclohexyl)benzonitril |
| 10 Gew.% | 1-[trans-4-(trans-4-Vinylcyclohexyl)cyclohexyläthinyl]-2,6-difluor-4-äthoxybenzol |
| Klp. (N/I): | 58,5°C, $V_{10}$ = 1,72 V, $t_{on}$ = 26 ms, $t_{off}$ = 47 ms, $\Delta n$ = 0,125 |

BM-10

| | |
|---|---|
| 80 Gew.% | 4-(trans-4-Pentyl-cyclohexyl)benzonitril |
| 20 Gew.% | 1-[trans-4-(trans-4-Vinylcyclohexyl)cyclohexyläthinyl]-2,6-difluor-4-äthoxybenzol |
| Klp. (N/I): | 62,5°C, $V_{10}$ = 1,82 V, $t_{on}$ = 30 ms, $t_{off}$ = 51 ms, $\Delta n$ = 0,129 |

BM-11

| | |
|---|---|
| 90 Gew.% | 4-(trans-4-Pentyl-cyclohexyl)benzonitril |
| 10 Gew.% | 1-{trans-4-[trans-4-(2-E-Chlorvinyl)cyclohexyl]cyclohexyläthinyl}-2,6-difluor-4-propylbenzol |
| Klp. (N/I): | 64,6°C, $V_{10}$ = 1,64 V, $t_{on}$ = 29 ms, $t_{off}$ = 45 ms, $\Delta n$ = 0,129 |

BM-12

| | |
|---|---|
| 80 Gew.% | 4-(trans-4-Pentyl-cyclohexyl)benzonitril |
| 20 Gew.% | 1-{trans-4-[trans-4-(2-E-Chlorvinyl)cyclohexyl]cyclohexyläthinyl}-2,6-difluor-4-propylbenzol |
| Klp. (N/I): | 75,4°C, $V_{10}$ = 1,73 V, $t_{on}$ = 32 ms, $t_{off}$ = 51 ms, $\Delta n$ = 0,135 |

BM-13

| | |
|---|---|
| 90 Gew.% | 4-(trans-4-Pentyl-cyclohexyl)benzonitril |
| 10 Gew.% | 1-{trans-4-[trans-4-(2-E-Chlorvinyl)cyclohexyl]cyclohexyläthinyl}-2,6-difluor-4-ethoxybenzol |
| Klp. (N/I): | 67,3°C, $V_{10}$ = 1,78 V, $t_{on}$ = 25 ms, $t_{off}$ = 43 ms, $\Delta n$ = 0,131 |

BM-14

80 Gew.% 4-(trans-4-Pentyl-cyclohexyl)benzonitril
20 Gew.% 1-{trans-4-[trans-4-(2-E-Chlorvinyl)cyclohexyl]cyclohexyläthinyl}-2,6-difluor-4-äthoxybenzol
Klp. (N/I): 81,3°C, $V_{10}$ = 1,87 V, $t_{on}$ = 28 ms, $t_{off}$ = 48 ms, $\Delta n$ = 0,140

BM-15

90 Gew.% 4-(trans-4-Pentyl-cyclohexyl)benzonitril
10 Gew.% 1-{trans-4-[2-(trans-4-Propylcyclohexyl)äthyl]cyclohexyläthinyl}-2,6-difluor-4-propylbenzol
Klp. (N/I): 61,2°C, $V_{10}$ = 1,72 V, $t_{on}$ = 25 ms, $t_{off}$ = 42 ms, $\Delta n$ = 0,123

BM-16

80 Gew.% 4-(trans-4-Pentyl-cyclohexyl)benzonitril
20 Gew.% 1-{trans-4-[2-(trans-4-Propylcyclohexyl)äthyl]cyclohexyläthinyl}-2,6-difluor-4-propylbenzol
Klp. (N/I): 68,4°C, $V_{10}$ = 1,85 V, $t_{on}$ = 28 ms, $t_{off}$ = 46 ms, $\Delta n$ = 0,126

BM-17

90 Gew.% 4-(trans-4-Pentyl-cyclohexyl)benzonitril
10 Gew.% 1-[trans-4-(3-Fluor-4-Chlorphenyl)cyclohexyläthinyl]-2,6-difluor-4-propylbenzol
Klp. (N/I): 55,2°C, $V_{10}$ = 1,50 V, $t_{on}$ = 30 ms, $t_{off}$ = 49 ms, $\Delta n$ = 0,127

BM-18

80 Gew.% 4-(trans-4-Pentyl-cyclohexyl)benzonitril
20 Gew.% 1-[trans-4-(3-Fluor-4-Chlorphenyl)cyclohexyläthinyl]-2,6-difluor-4-propylbenzol
Klp. (N/I): 56,4°C, $V_{10}$ = 1,44 V, $t_{on}$ = 34 ms, $t_{off}$ = 55 ms, $\Delta n$ = 0,133

BM-19

90 Gew.% 4-(trans-4-Pentyl-cyclohexyl)benzonitril
10 Gew.% 1-[trans-4-(3,4-Difluorphenyl)cyclohexyläthinyl]-2,6-difluor-4-äthylbenzol
Klp. (N/I): 53,1°C, $V_{10}$ = 1,50 V, $t_{on}$ = 27 ms, $t_{off}$ = 47 ms, $\Delta n$ = 0,121

BM-20

80 Gew.% 4-(trans-4-Pentyl-cyclohexyl)benzonitril
20 Gew.% 1-[trans-4-(3,4-Difluorphenyl)cyclohexyläthinyl]-2,6-difluor-4-äthylbenzol
Klp. (N/I): 51,7°C, $V_{10}$ = 1,40 V, $t_{on}$ = 30 ms, $t_{off}$ = 54 ms, $\Delta n$ = 0,124

BM-21

90 Gew.% 4-(trans-4-Pentyl-cyclohexyl)benzonitril
10 Gew.% 1-[trans-4-(3,4-Difluorphenyl)cyclohexyläthinyl]-2,6-difluor-4-propylbenzol
Klp. (N/I): 53,1°C, $V_{10}$ = 1,49 V, $t_{on}$ = 29 ms, $t_{off}$ = 51 ms, $\Delta n$ = 0,123

BM-22

80 Gew.% 4-(trans-4-Pentylcyclohexyl)benzonitril
20 Gew.% 1-[trans-4-(3,4-Difluorphenyl)cyclohexyläthinyl]-2,6-difluor-4-propylbenzol
Klp. (N/I): 52,0°C, $V_{10}$ = 1,37 V, $t_{on}$ = 33 ms, $t_{off}$ = 54 ms, $\Delta n$ = 0,125

BM-23

90 Gew.% 4-(trans-4-Pentyl-cyclohexyl)benzonitril
10 Gew.% 1-[trans-4-(3,4-Difluorophenyl)cyclohexyläthinyl]-2,6-difluor-4-butylbenzol
Klp. (N/I): 52,3°C, $V_{10}$ = 1,52 V, $t_{on}$ = 27 ms, $t_{off}$ = 48 ms, $\Delta n$ = 0,120

BM-24

| | |
|---|---|
| 80 Gew.% | 4-(trans-4-Pentyl-cyclohexyl)benzonitril |
| 20 Gew.% | 1-[trans-4-(3,4-Difluorphenyl)cyclohexyläthinyl]-2,6-difluor-4-butylbenzol |
| Klp. (N/I): | 50,5°C, $V_{10}$ = 1,40 V, $t_{on}$ = 33 ms, $t_{off}$ = 57 ms, $\Delta n$ = 0,123 |

BM-25

| | |
|---|---|
| 90 Gew.% | 4-(trans-4-Pentyl-cyclohexyl)benzonitril |
| 10 Gew.% | 1-[trans-4-(3,4,5-Trifluorphenyl)cyclohexyläthinyl]-2,6-difluor-4-äthylbenzol |
| Klp. (N/I): | 51,2°C, $V_{10}$ = 1,45 V, $t_{on}$ = 27 ms, $t_{off}$ = 48 ms, $\Delta n$ = 0,121 |

BM-26

| | |
|---|---|
| 80 Gew.% | 4-(trans-4-Pentyl-cyclohexyl)benzonitril |
| 20 Gew.% | 1-[trans-4-(3,4,5-Trifluorphenyl)cyclohexyläthinyl]-2,6-difluor-4-äthylbenzol |
| Klp. (N/I): | 47,6°C, $V_{10}$ = 1,30 V, $t_{on}$ = 31 ms, $t_{off}$ = 58 ms, $\Delta n$ = 0,120 |

BM-27

| | |
|---|---|
| 90 Gew.% | 4-(trans-4-Pentyl-cyclohexyl)benzonitril |
| 10 Gew.% | 1-[trans-4-(3,4,5-Trifluorophenyl)cyclohexyläthinyl]-2,6-difluor-4-propylbenzol |
| Klp. (N/I): | 51,3°C, $V_{10}$ = 1,40 V, $t_{on}$ = 31 ms, $t_{off}$ = 51 ms, $\Delta n$ = 0,122 |

BM-28

| | |
|---|---|
| 80 Gew.% | 4-(trans-4-Pentyl-cyclohexyl)benzonitril |
| 20 Gew.% | 1-[trans-4-(3,4,5-Trifluorophenyl)cyclohexyläthinyl]-2,6-difluor-4-propylbenzol |
| Klp. (N/I): | 48,0°C, $V_{10}$ = 1,24 V, $t_{on}$ = 37 ms, $t_{off}$ = 60 ms, $\Delta n$ = 0,120 |

BM-29

| | |
|---|---|
| 90 Gew.% | 4-(trans-4-Pentyl-cyclohexyl)benzonitril |
| 10 Gew.% | 1-[trans-4-(3,4,5-Trifluorphenyl)cyclohexyläthinyl]-2,6-difluor-4-butylbenzol |
| Klp. (N/I): | 50,7°C, $V_{10}$ = 1,44 V, $t_{on}$ = 29 ms, $t_{off}$ = 52 ms, $\Delta n$ = 0,120 |

BM-30

| | |
|---|---|
| 80 Gew.% | 4-(trans-4-Pentyl-cyclohexyl)benzonitril |
| 20 Gew.% | 1-[trans-4-(3,4,5-Trifluorophenyl)cyclohexyläthinyl]-2,6-difluor-4-butylbenzol |
| Klp. (N/I): | 56,8°C, $V_{10}$ = 1,29 V, $t_{on}$ = 33 ms, $t_{off}$ = 62 ms, $\Delta n$ = 0,116 |

BM-31

| | |
|---|---|
| 90 Gew.% | 4-(trans-4-Pentyl-cyclohexyl)benzonitril |
| 10 Gew.% | 1-[trans-4-(3,5-Difluor-4-chlorphenyl)cyclohexyläthinyl]-2,6-difluor-4-propylbenzol |
| Klp. (N/I): | 53,3°C, $V_{10}$ = 1,52 V, $t_{on}$ = 29 ms, $t_{off}$ = 50 ms, $\Delta n$ = 0,124 |

BM-32

| | |
|---|---|
| 80 Gew.% | 4-(trans-4-Pentyl-cyclohexyl)benzonitril |
| 20 Gew.% | 1-[trans-4-(3,5-Difluor-4-chlorphenyl)cyclohexyläthinyl]-2,6-difluor-4-propylbenzol |
| Klp. (N/I): | 52,3°C, $V_{10}$ = 1,41 V, $t_{on}$ = 34 ms, $t_{off}$ = 61 ms, $\Delta n$ = 0,127 |

BM-33

| | |
|---|---|
| 90 Gew.% | 4-(trans-4-Pentyl-cyclohexyl)benzonitril |
| 10 Gew.% | 1-[trans-4-(3,5-Difluor-4-trifluormethylphenyl)cyclohexyläthinyl]-2,6-difluor-4-propylbenzol |
| Klp. (N/I): | 50,6°C, $V_{10}$ = 1,43 V, $t_{on}$ = 29 ms, $t_{off}$ = 53 ms, $\Delta n$ = 0,121 |

BM-34

80 Gew.% 4-(trans-4-Pentyl-cyclohexyl)benzonitril
20 Gew.% 1-[trans-4-(3,5-Difluor-4-trifluormethylphenyl)cyclohexyläthinyl]-2,6-difluor-4-propylbenzol
Klp. (N/I): 46,9°C, $V_{10}$ = 1,28 V, $t_{on}$ = 35 ms, $t_{off}$ = 67 ms $\Delta n$ = 0,122

**Patentansprüche**

1. Verbindungen der allgemeinen Formel

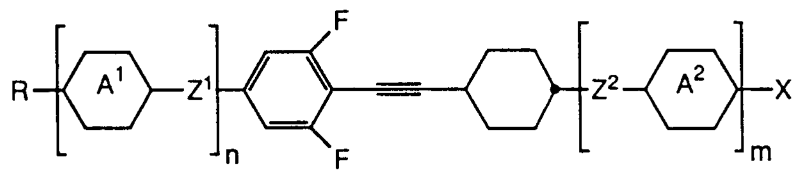

I

worin

R Alkyl, Alkoxy, Alkenyl oder Alkenyloxy mit 1 bzw. 2 bis 12 Kohlenstoffatomen bedeutet, in welchen eine $CH_2$-Gruppe durch Sauerstoff und/oder eines oder mehrere Wasserstoffatome durch Fluoratome ersetzt sein können, mit der Massgabe, dass zwei Sauerstoffatome nicht direkt benachbart sind;
$A^1$ 1,4-Phenylen, Pyridin-2,5-diyl, Pyrimidin-2,5-diyl, trans-1,4-Cyclohexylen oder trans 1,3-Dioxan-2,5-diyl darstellt;
$A^2$ trans-1,4-Cyclohexylen oder gegebenenfalls mit Fluor substituiertes 1,4-Phenylen bedeutet;
$Z^1$ eine einfache Kovalenzbindung, $-CH_2CH_2-$, $-OCH_2-$, $-CH_2O-$, $-(CH_2)_4-$, $-O(CH_2)_3-$, $-(CH_2)_3O-$ oder, falls Ring $A^1$ einen gesättigten Ring darstellt, auch die trans-Form von $-CH=CH(CH_2)_2-$ oder $-CH=CHCH_2O-$ bedeutet;
$Z^2$ eine einfache Kovalenzbindung, $-CH_2CH_2-$, $-OCH_2-$, $-CH_2O-$, $-(CH_2)_4-$, $-O(CH_2)_3-$, $-(CH_2)_3O-$, oder die trans-Form von $-CH=CH(CH_2)_2-$ oder $-CH=CHCH_2O-$ bedeutet;
n 0 oder 1 bedeutet;
m 0 oder 1 bedeutet, mit der Massgabe, dass $n+m \leq 1$ ist; und
X Cyano, $-CF_3$, $-OCF_3$, $-OCHF_2$, $-CH=CF_2$, Alkyl, Alkenyl, Alkoxy, Alkenyloxy oder Alkoxyalkyl mit 1 bzw. 2 bis 6 Kohlenstoffatomen, oder an trans-1,4-Cyclohexylen auch -CH=CHF oder -CH=CHCl, oder an 1,4-Phenylen auch Fluor oder Chlor bedeutet.

2. Verbindungen der allgemeinen Formel I gemäss Anspruch 1, dadurch gekennzeichnet, dass $Z^1$ eine einfache Kovalenzbindung, $-CH_2CH_2-$, $-CH_2O-$ oder $-OCH_2-$ bedeutet, vorzugsweise eine einfache Kovalenzbindung oder $-CH_2CH_2-$.

3. Verbindungen der allgemeinen Formel I gemäss Anspruch 1, dadurch gekennzeichnet, dass $Z^2$ eine einfache Kovalenzbindung oder $-CH_2CH_2-$ bedeutet.

4. Verbindungen gemäss einem der Ansprüche 1 oder 3 der allgemeinen Formeln

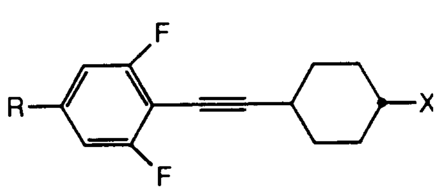

I-1

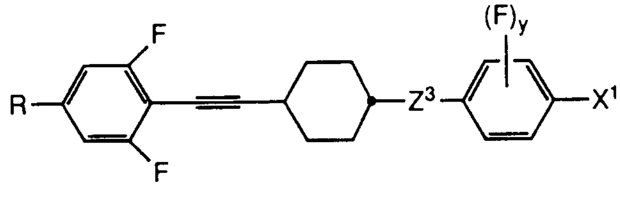

I-2

worin

R Alkyl, Alkoxy, Alkenyl oder Alkenyloxy mit 1 bzw. 2 bis 6 Kohlenstoffatomen bedeutet, in welchen eine $CH_2$-Gruppe durch Sauerstoff ersetzt sein kann, mit der Massgabe, dass zwei Sauerstoffatome nicht direkt benachbart sind;

X $-OCF_3$, $-OCHF_2$, $-CH=CF_2$, -CH=CHF, -CH=CHCl, Alkyl, Alkenyl, Alkoxy oder Alkenyloxy mit 1 bzw. 2 bis 3 Kohlenstoffatomen bedeutet;

$X^1$ Fluor, Chlor, Cyano, $-OCF_3$, $-OCHF_2$ oder $-CH=CF_2$ bedeuten;

$Z^3$ eine einfache Kovalenzbindung oder $-CH_2CH_2-$ bedeutet; und

y 0, 1 oder 2 bedeutet; und der Phenylring

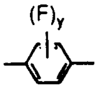

unsubstituiert oder wie folgt substituiert ist

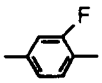 , 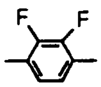 oder 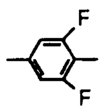 .

5. Verbindungen gemäss Anspruch 4, dadurch gekennzeichnet, dass $X^1$ Fluor, Chlor oder Cyano bedeutet.

6. Verbindungen gemäss einem der Ansprüche 1 oder 2 der allgemeinen Formeln

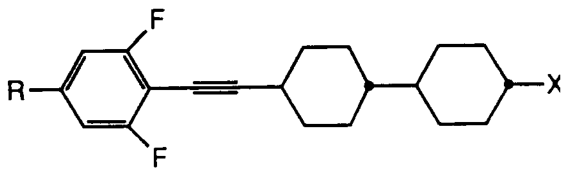

I-3

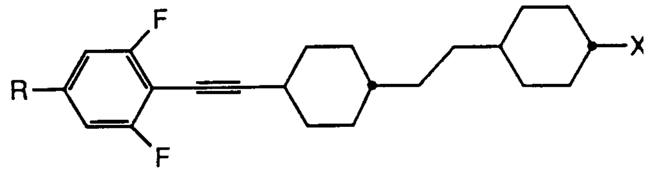

I-4

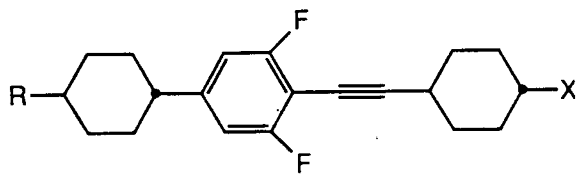

I-5

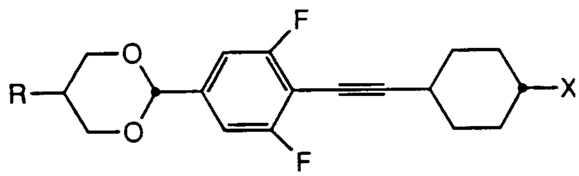

I-6

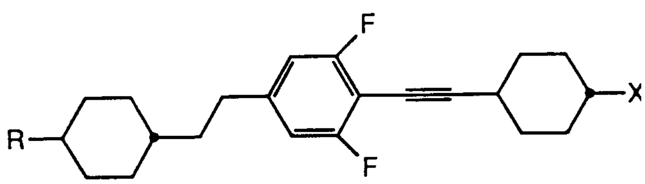

I-7

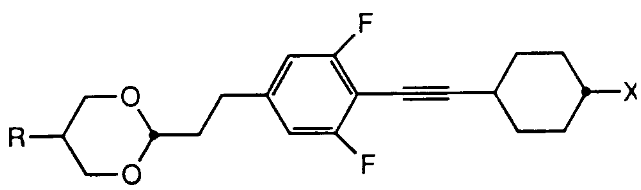

I-8

worin

R Alkyl, Alkoxy, Alkenyl oder Alkenyloxy mit 1 bzw. 2 bis 6 Kohlenstoffatomen bedeutet, in welchen eine $CH_2$-Gruppe durch Sauerstoff ersetzt sein kann, mit der Massgabe, dass zwei Sauerstoffatome nicht direkt benachbart sind;

X $-OCF_3$, $-OCHF_2$, $-CH=CF_2$, -CH=CHF, -CH=CHCl, Alkyl, Alkenyl, Alkoxy oder Alkenyloxy mit 1 bzw. 2 bis 3 Kohlenstoffatomen bedeutet

**7.** Verbindungen gemäss Anspruch 6, dadurch gekennzeichnet, dass X Methyl, Aethyl, Propyl, Vinyl, 1E-Propenyl bedeutet, oder aber $-CH=CF_2$, -CH=CHF oder -CH=CHCl darstellt.

**8.** Flüssigkristalline Gemische enthaltend mindestens zwei Komponenten, dadurch gekennzeichnet, dass mindestens eine Komponente eine Verbindung der in Anspruch 1 definierten Formel I ist.

**9.** Flüssigkristalline Gemische gemäss Anspruch 8, dadurch gekennzeichnet, dass der Anteil an Verbindungen der in Anspruch 1 definierten Formel I 3-40 Gew.-% beträgt.

**10.** Flüssigkristalline Gemische gemäss einem der Ansprüche 8 oder 9, dadurch gekennzeichnet, dass der Anteil an Verbindungen der in Anspruch 1 definierten Formel I 5-30 Gew.-% beträgt.

**11.** Verwendung der in Anspruch 1 definierten Verbindungen der Formel I für elektro-optische Zwecke.

## Claims

1. Compounds of the general formula

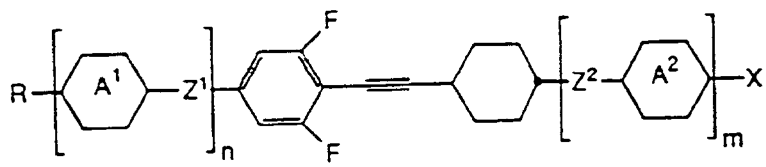

I

in which

- R is alkyl, alkoxy, alkenyl or alkenyloxy having 1 or 2 to 12 carbon atoms in which one $CH_2$ group may be replaced by oxygen and/or one or more hydrogen atoms may be replaced by fluorine atoms, with the proviso that two oxygen atoms are not directly adjacent;
- $A^1$ is 1,4-phenylene, pyridine-2,5-diyl, pyrimidine-2,5-diyl, trans-1,4-cyclohexylene or trans-1,3-dioxane-2,5-diyl;
- $A^2$ is trans-1,4-cyclohexylene or optionally fluorine-substituted 1,4-phenylene;
- $Z^1$ is a single covalent bond, $-CH_2CH_2-$, $-OCH_2-$, $-CH_2O-$, $-(CH_2)_4-$, $-O(CH_2)_3-$, $-(CH_2)_3O-$ or, if ring $A^1$ is a saturated ring, $Z^1$ is alternatively the trans form of $-CH=CH(CH_2)_2-$ or $-CH=CHCH_2O-$;
- $Z^2$ is a single covalent bond, $-CH_2CH_2-$, $-OCH_2-$, $-CH_2O-$, $-(CH_2)_4-$, $-O(CH_2)_3-$, $-(CH_2)_3O-$, or the trans form of $- CH=CH(CH_2) -$ or $-CH=CHCH_2O-$;
- n is 0 or 1;
- m is 0 or 1, with the proviso that n+m is $\leq 1$; and
- X is cyano, $-CF_3$, $-OCF_3$, $-OCHF_2$, $-CH=CF_2$, alkyl, alkenyl, alkoxy, alkenyloxy or alkoxyalkyl having 1 or 2 to 6 carbon atoms, or, on trans-1,4-cyclohexylene, X is alternatively -CH=CHF or -CH=CHCl, or, on 1,4-phenylene, X is alternatively fluorine or chlorine.

2. Compounds of the general formula I according to Claim 1, characterized in that $Z^1$ is a single covalent bond, $-CH_2CH_2-$, $-CH_2O-$ or $-OCH_2-$, preferably a single covalent bond or $-CH_2CH_2-$.

3. Compounds of the general formula I according to Claim 1, characterized in that $Z^2$ is a single covalent bond or $-CH_2CH_2-$.

4. Compounds according to one of Claims 1 and 3, of the general formulae

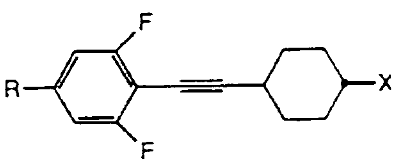

I-1

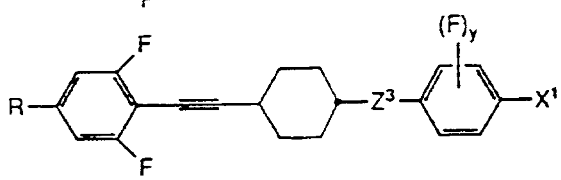

I-2

in which

- R is alkyl, alkoxy, alkenyl or alkenyloxy having 1 or 2 to 6 carbon atoms in which one $CH_2$ group may be replaced by oxygen, with the proviso that two oxygen atoms are not directly adjacent;
- X is $-OCF_3$, $-OCHF_2$, $-CH=CF_2$, -CH=CHF, -CH=CHCl, alkyl, alkenyl, alkoxy or alkenyloxy having 1 or 2 to 3 carbon atoms;
- $X^1$ is fluorine, chlorine, cyano, $-OCF_3$, $-OCHF_2$ or $-CH=CF_2$;
- $Z^3$ is a single covalent bond or $-CH_2CH_2-$; and
- y is 0, 1 or 2; and the phenyl ring

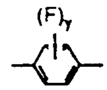

is unsubstituted or substituted as follows

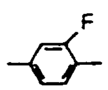 , 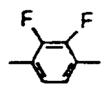 or 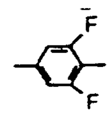 .

5. Compounds according to Claim 4, characterized in that $X^1$ is fluorine, chlorine or cyano.

6. Compounds according to one of Claims 1 and 2, of general formulae

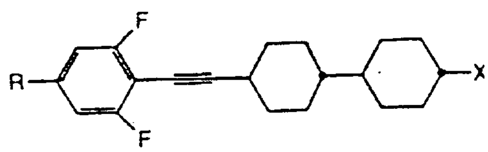

I-3

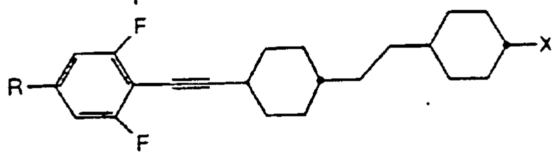

I-4

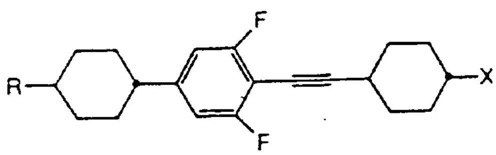

I-5

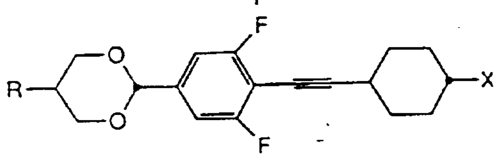

I-6

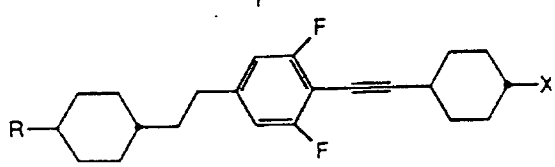

I-7

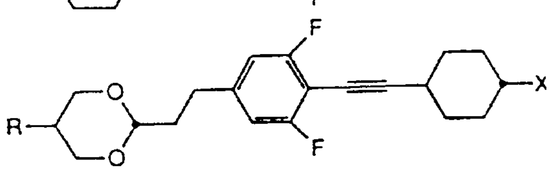

I-8

in which

R is alkyl, alkoxy, alkenyl or alkenyloxy having 1 or 2 to 6 carbon atoms in which one $CH_2$ group may be replaced by oxygen, with the proviso that two oxygen atoms are not directly adjacent;

X is $-OCF_3$, $-OCHF_2$, $-CH=CF_2$, -CH=CHF, -CH=CHCl, alkyl, alkenyl, alkoxy or alkenyloxy having 1 or 2 to 3 carbon atoms.

7. Compounds according to Claim 6, characterized in that X is methyl, ethyl, propyl, vinyl, 1E-propenyl or alternatively

$-CH=CF_2$, -CH=CHF or -CH=CHCl.

**8.** Liquid-crystalline mixtures comprising at least two components, characterized in that at least one component is a compound of the formula I defined in Claim 1.

**9.** Liquid-crystalline mixtures according to Claim 8, characterized in that the proportion of compounds of the formula I defined in Claim 1 is 3-40% by weight.

**10.** Liquid-crystalline mixtures according to one of Claims 8 and 9, characterized in that the proportion of compounds of the formula I defined in Claim 1 is 5-30% by weight.

**11.** Use of the compounds of the formula I defined in Claim 1 for electro-optical purposes.

**Revendications**

**1.** Composés de formule générale

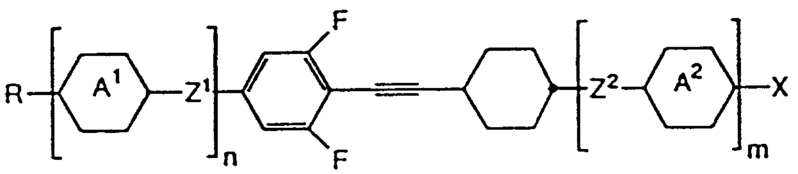

I

dans laquelle

R est un groupe alkyle, alcoxy, alcényle ou alcényloxy ayant de 1 ou 2 à 12 atomes de carbone, où un groupe $CH_2$ peut être remplacé par un oxygène, et/ou un ou plusieurs atomes d'hydrogène peuvent être remplacé par des atomes de fluor, à la condition que deux atomes d'oxygène ne soient pas directement voisins ;
$A^1$ est le radical 1,4-phénylène, pyridine-2,5-diyle, pyrimidine-2,5-diyle, trans-1,4-cyclohexylène ou trans-1,3-dioxanne-2,5-diyle ;
$A^2$ est le radical trans-1,4-cyclohexylène ou un radical 1,4-phénylène éventuellement fluoré ;
$Z^1$ est une liaison covalente simple, $-CH_2CH_2-$, $-OCH_2-$, $-CH_2O-$, $-(CH_2)_4-$, $-O(CH_2)_3-$, $-(CH_2)_3O$ ou encore, si le noyau $A^1$ est un noyau saturé, représente aussi la forme trans de $-CH=CH(CH_2)_2-$ ou de $-CH=CHCH_2O-$ ;
$Z^2$ est une liaison covalente simple, $-CH_2CH_2-$, $-OCH_2-$, $-CH_2O-$, $-(CH_2)_4-$, $-O(CH_2)_3-$, $-(CH_2)_3O$, ou encore la forme trans de $-CH=CH(CH_2)_2-$ ou de $-CH=CHCH_2O-$ ;
n vaut 0 ou 1 ;
m vaut 0 ou 1, à la condition que $n + m \leq 1$ ; et
X est le groupe cyano, $-CF_3$, $-OCF_3$, $-OCHF_2$, $-CH=CF_2$, un groupe alkyle, alcényle, alcoxy, alcényloxy ou alcoxyalkyle ayant de 1 ou 2 à 6 atomes de carbone, ou encore, sur le trans-1,4-cyclohexylène, représente aussi -CH=CHF ou -CH=CHCl, ou encore, sur le 1,4-phénylène, représente aussi le fluor ou le chlore.

**2.** Composés de formule générale I selon la revendication 1, caractérisés en ce que $Z^1$ est une liaison covalente simple, $-CH_2CH_2-$, $-CH_2O-$ ou $-OCH_2-$, de préférence une liaison covalente simple ou $-CH_2CH_2-$.

**3.** Composés de formule générale I selon la revendication 1, caractérisés en ce que $Z^2$ est une liaison covalente simple ou $-CH_2CH_2-$.

**4.** Composés selon l'une des revendications 1 ou 3, de formules générales

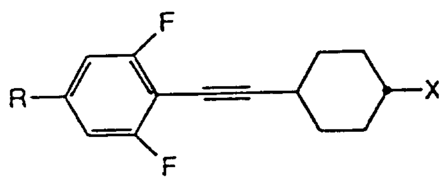

I-1

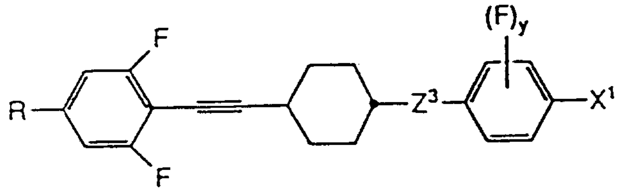

I-2

dans lesquelles

R est un groupe alkyle, alcoxy, alcényle ou alcényloxy ayant de 1 ou 2 à 6 atomes de carbone, où un groupe $CH_2$ peut être remplacé par un oxygène, à la condition que deux atomes d'oxygène ne soient pas directement voisins ;
X est $-OCF_3$, $-OCHF_2$, $-CH=CF_2$, -CH=CHF, -CH=CHCl, un groupe alkyle, alcényle, alcoxy ou alcényloxy ayant de 1 ou 2 à 3 atomes de carbone ;
$X^1$ est le fluor, le chlore ou le groupe cyano, $-OCF_3$, $-OCHF_2$ ou $-CH=CF_2$ ;
$Z^3$ est une liaison covalente simple ou $-CH_2CH_2-$ ; et
y vaut 0, 1 ou 2 ; et le noyau phényle

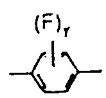

est non-substitué, ou est substitué comme suit :

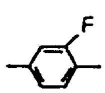 , 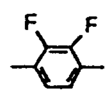 ou 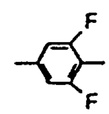 .

5. Composés selon la revendication 4, caractérisés en ce que $X^1$ est le fluor, le chlore ou le groupe cyano.

6. Composés selon l'une des revendications 1 ou 2, de formules générales

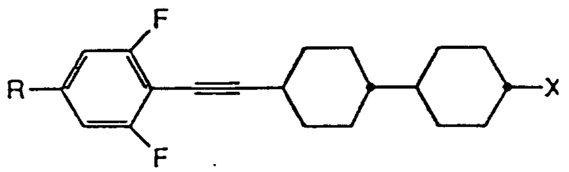

I-3

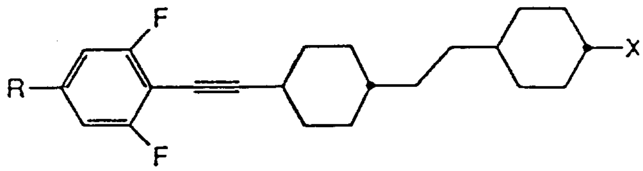

I-4

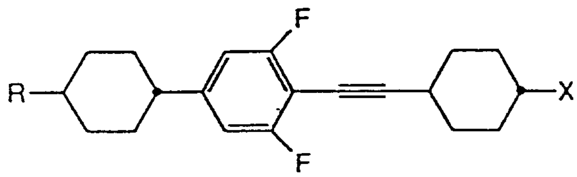

I-5

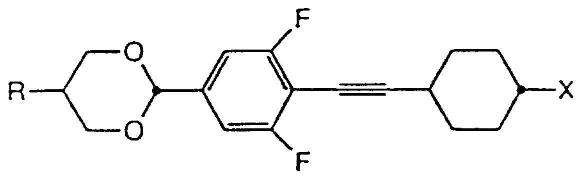

I-6

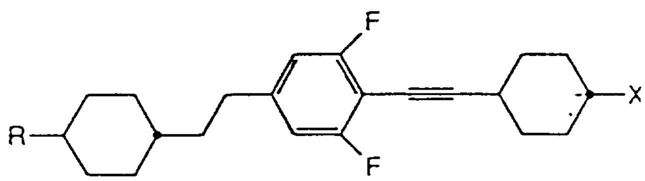

I-7

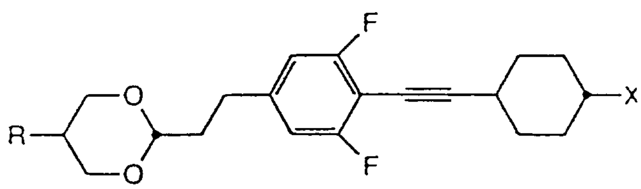

I-8

dans lesquelles

R est un groupe alkyle, alcoxy, alcényle ou alcényloxy ayant de 1 ou 2 à 6 atomes de carbone, où un groupe $CH_2$ peut être remplacé par un oxygène, à la condition que deux atomes d'oxygène ne soient pas directement voisins ;
X est $-OCF_3$, $-OCHF_2$, $-CH=CF_2$, -CH=CHF, -CH=CHCl, un groupe alkyle, alcényle, alcoxy ou alcényloxy ayant de 1 ou 2 à 3 atomes de carbone.

**7.** Composés selon la revendication 6, caractérisés en ce que X est le radical méthyle, éthyle, propyle, vinyle, 1E-propényle, ou encore représente $-CH=CF_2$, -CH=CHF ou -CH=CHCl.

**8.** Mélanges mésomorphes contenant au moins deux constituants, caractérisés en ce qu'au moins un constituant est un composé de formule I selon la revendication 1.

**9.** Mélanges mésomorphes selon la revendication 8, caractérisés en ce que la proportion des composés de formule I selon la revendication 1 est de 3 à 40 % en poids.

**10.** Mélanges mésomorphes selon l'une des revendications 8 ou 9, caractérisés en ce que la proportion des composés de formule I selon la revendication 1 est de 5 à 30 % en poids.

**11.** Utilisation des composés selon la revendication 1, de formule I, pour des applications électro-optiques.